# EUROPEAN PATENT APPLICATION

(11) **EP 1 367 123 A1**
(43) Date of publication of application: **03.12.2003**
(21) Application number: 02710489.2
(22) Date of filing: 04.02.2002
(51) Int. Cl.: C12N 15/12

(54) **NEUROTONIN AND USE THEREOF**

(30) Priority: 09.02.2001 JP 2001034217
(71) Applicant: Locomogene, Inc., Tokyo 150-0002 (JP)
(72) Inventor: NAKAJIMA, Toshihiro;, Yokohama-shi, Kanagawa 224 -0001 (JP)
(74) Representative: Grant, Anne Rosemary
(86) International application number: JP0200885
(87) International publication number: WO02064787

(57) **Abstract**

To specify a substance causing an overexcitation of an Isaacs syndrome and to establish an effective diagnosing and treating method, an immnoscreening method was applied to a serum obtained from an Isaacs syndrome patient. A specific cDNA was separated from a cDNA library derived from a nerve cell. The nucleotide sequence was elucidated and a novel protein, i.e., a neurotonin was obtained by cloning based thereon. The novel protein of which amino acid sequence was also determined is regarded as a key substance of the Isaacs syndrome and is useful for the studies of peripheral nerve lesions and a clinical application.

## Description

### Technical Field

The present invention relates to neurotonin and utilization thereof. More specifically, the present invention relates to a neurotonin, a cDNA for encoding the neurotonin, a related nucleic acid molecule, a monoclonal antibody to the neurotonin and the like, and utilization thereof.

### Background Art

Peripheral nerve lesions result from the defect of an excitation transmission of a nerve in the neuromuscular junction terminals of a motor nerve and often cause a myopathy. For this reason, there have been known various disease conditions, for example, a sensation dysesthesia, motion disorders, autonomic nerve lesions and the like. The cause of the diseases is also diversified and, in detail, is not known in many cases. An Isaacs syndrome has been known as a neuropathy and myopathy causing the VGKC (Voltage gated potassium channel) abnormality of nerve terminals and the continuous discharge of acetylcholine from the nerve terminals, and has also been referred to as a syndrome of continuous muscle-fiber activity. As a clinical feature, there have been observed symptoms, for example, relaxation difficulties caused after muscular contraction such as the muscle rigidity of limbs, dolorous muscular convulsion and limited finger abduction, dysbasia, hyperhidrosis and the like. Although the mechanism of the symptoms is not known, the presence of a complication of a thymoma and a case in which a blood contains an immune complex are observed. Consequently, the presence of an autoimmune disease can also be supposed.

Under actual circumstances, there has not been a specific and effective method of treating peripheral nerve lesions including the Isaacs syndrome, and the treatment is limited to a countermeasure therapy, for example, a treating method using a plasma exchange, administration of vitamin B₁₂ and the like. Since the arrival of a real aging society is close at hand, there has been ardently desired a method of effectively carrying out a treatment for peripheral multiple neurosis to be a complication of diabetes, the whole painful numbness of a muscle and the like in addition to the peripheral nerve lesions.

The present inventor grasped the fact that a cDNA isolated from an Isaacs syndrome patient encodes a certain specific protein and the protein is increased in the blood of the patient. As a result of intensive studies, the present inventor identified the cDNA and the protein and ascertained that they are factors related to the disease conditions, and determined their sequence structure and thus completed the present invention related to a diagnosis and treatment utilizing the nucleic acid molecules and protein. The DNA molecule and the protein according to the present invention provide various substances and methods which contribute to basic studies related to peripheral nerve lesions and clinical applications.

### Object of the Invention

It is an object of the present invention, on the basis of the structure and function of neurotonin or cDNA coding therefor, to provide a nucleic acid molecule, a monoclonal antibody to a neurotonin, clinical testing chemicals, a remedy, a novel vector and a blood filtering material conjugating an antineurotonin antibody, a culture medium, transgenic animals, animals for screening and the like by utilizing them.

### Summary of the Invention

The present invention provides a peripheral nerve cell protein having the following properties (which will be hereinafter referred to as a "neurotonin"),
wherein the protein
a) is found in peripheral nerve cells of Isaacs syndrome patients or other patients having peripheral nerve lesions,
b) reacts with an antibody found in a blood of the patients,
c) causes a similar symptom to an Isaacs syndrome group with an immunization to a rabbit, and
d) has a molecular weight of approximately 66 kDa according to SDS-PAGE. The DNA according to the present invention is a cDNA encoding the neurotonin.

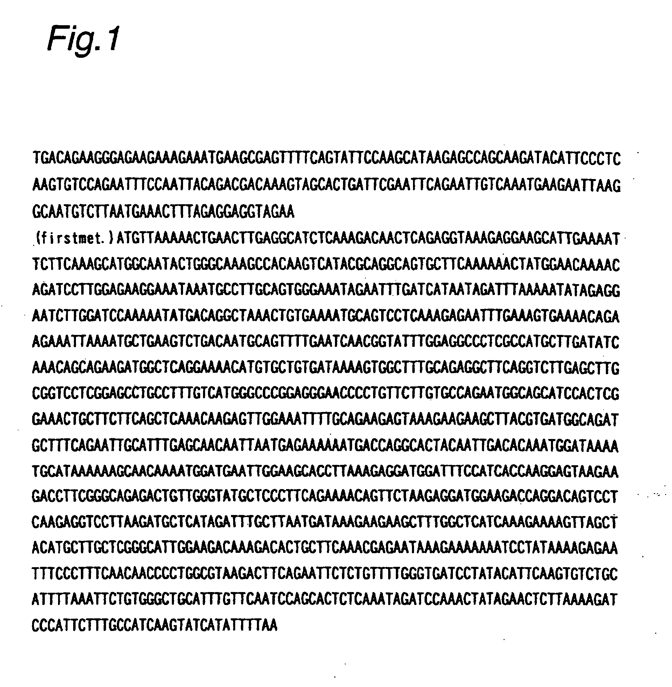

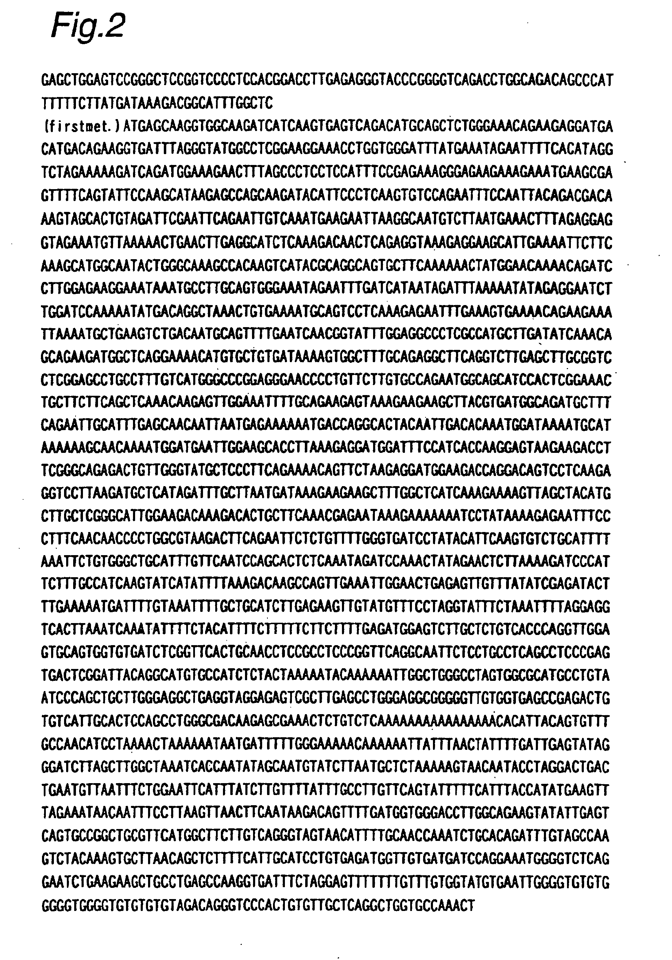

Furthermore, the DNA according to the present invention is a DNA encoding the neurotonin or including all nucleotide sequences shown in Fig. 1 or 2.

The present invention also includes a nucleic acid molecule encoding a neurotonin, including at least one nucleotide sequence substantially corresponding to a whole or part of the nucleotide sequences shown in Fig. 1 or 2, or including a sequence which is substantially homologous with any of the sequences or is hybridized with any of the sequences.

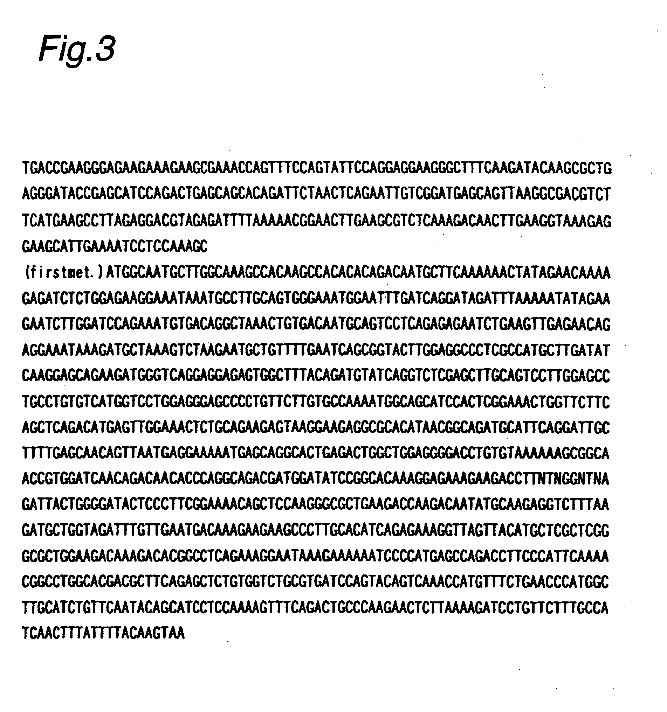

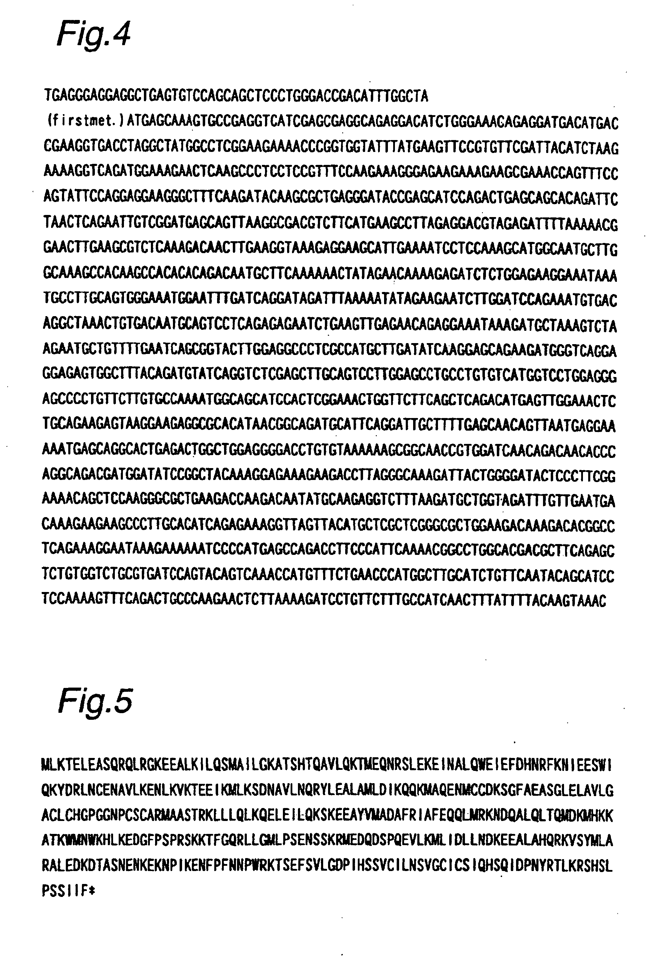

The DNA according to the present invention also includes a DNA encoding a mouse neurotonin or containing all nucleotide sequences shown in Fig. 3 or 4. Moreover, a mouse cDNA encoding the mouse neurotonin also belongs to the present invention.

Moreover, the present invention also includes a nucleic acid molecule encoding a mouse neurotonin, including at least one nucleotide sequence substantially corresponding to a whole or part of the nucleotide sequences shown in Fig. 3 or 4, or including a sequence encoding the mouse neurotonin which is substantially homologous with any of the sequences or is hybridized with any of the sequences.

Furthermore, the present invention also includes an antisense RNA or an antisense DNA to the cDNA or any of the nucleic acid molecules, or a ribozyme recognizing and cleaving said cDNA, said nucleic acid molecule or an RNA transcribed from a part thereof.

An expression vector, a cloning vector or a cosmid according to the present invention includes the cDNA or any of said nucleic acid molecules.

Atransformant according to the present invention retains the vector or the cosmid.

A prokaryotic cell, a eukaryotic cell or a variant cell according to the present invention contains any of the nucleic acid molecules described above.

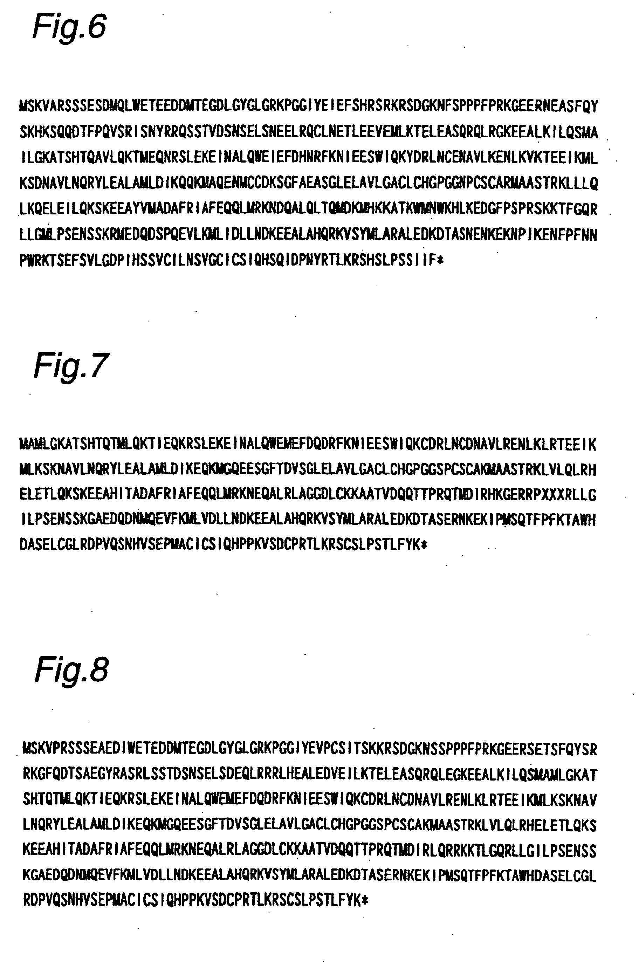

A neurotonin according to the present invention has an amino acid sequence shown in Fig. 5 or 6.

A protein according to the present invention has an activity to bind to an antibody found in a blood of the patient and the following feature a) or b).
a) A polypeptide such as a polypeptide having an amino acid sequence shown in Fig. 5 or 6, a polypeptide having an amino acid sequence in which at least one amino acid i's deleted, substituted or added and/or inserted in the amino acid sequence shown in Fig. 5 or 6, a synthetic polypeptide including an amino acid sequence constituting an antigenic portion of a neurotonin, functionally equivalent variants thereof, or a fused polypeptide further including the aforesaid amino acid sequences, and
b) a protein encoded by a DNA to be hybridizable with the DNA having a nucleotide sequence shown in any of Figs. 1 to 4.

Moreover, a protein according to the present invention can bind to a "14-3-3" protein existing in a peripheral nerve cell and has the following feature a) or b).
a) A polypeptide such as a polypeptide having an amino acid sequence shown in Fig. 5 or 6, a polypeptide having an amino acid sequence in which at least one amino acid is deleted, substituted or added and/or inserted in the amino acid sequence shown in Fig. 5 or 6, a synthetic polypeptide including an amino acid sequence constituting an antigenic portion of a neurotonin, functionally equivalent variants thereof, or a fused polypeptide further including the aforesaid amino acid sequences, and
b) a protein encoded by a DNA to be hybridizable with. the DNA having a nucleotide sequence shown in any of Figs. 1 to 4.

Furthermore, the present invention includes an immunologically active domain of the protein or a fragment having the domain.

A polypeptide according to the present invention also includes a polypeptide having an amino acid sequence shown in Fig. 7 or 8, a polypeptide having an amino acid sequence in which at least one amino acid is deleted, substituted or added and/or inserted in the amino acid sequence shown in Fig. 7 or 8, a synthetic polypeptide including an amino acid sequence constituting an antigenic portion of a neurotonin, functionally equivalent variants thereof or a fused polypeptide further including the aforesaid amino acid sequences.

A reagent according to the present invention comprising any of the aforesaid proteins for an immunological analysis serves to assay an antibody recognizing the protein.

Moreover, said reagent for an immunological analysis is used to assess a diagnosis or treatment effect of an Isaacs syndrome or other peripheral nerve lesions.

A reagent according to the present invention comprising an antibody to react to any of the aforesaid proteins for an immunological analysis serves to analyze the protein.

Moreover, the reagent for an immunological analysis is used to assess a diagnosis or treatment effect of an Isaacs syndrome or other peripheral nerve lesions.

In the reagent for an immunological analysis, furthermore, any of the proteins to be analyzed is present in peripheral nerve cells.

The present invention provides a neurotonin detecting method of analyzing an antibody which is present in a body fluid of a subject and reacts with any of the proteins.

A method of screening a ligand bind to the protein according to the present invention comprises the following steps:
a) causing a candidate compound of the ligand to come in contact with the protein described above; and
b) selecting a candidate compound having a binding activity to the protein.

The present invention provides an assay method of measuring any of the proteins on the basis of an affinity of an antiligand and the ligand obtainable by the screening method by using, as the antiligand, the protein.

A screening method according to the present invention for a compound inhibiting a binding of any of the proteins to the ligand and comprises the following steps:
a) causing the protein to come in contact with the ligand under the presence of a candidate compound; and
b) selecting a candidate compound having an activity to inhibit the binding of the protein to the ligand.

A compound according to the present invention also includes a compound which can be obtained by the screening method.

A neurotonin inhibitor according to the present invention comprises a compound which can be obtained by the screening method.

A medicine for treating peripheral nerve lesions according to the present invention comprises a compound which can be obtained by the screening method.

An antibody according to the present invention is an antibody capable of binding to any of the proteins..

The antibody includes a monoclonal antibody. Another monoclonal antibody according to the present invention is a monoclonal antibody against an antibody causing peripheral nerve lesions.

A method according to the present invention includes a method of detecting or separating a cell expressing any of the aforesaid proteins by setting, as a marker, the protein or an expression of a gene encoding the protein. This method includes the case in which the cell is a peripheral nerve cell.

A reagent for detecting or separating a cell expressing any of the proteins according to the present invention comprises the antibody described above.

A reagent according to the present invention comprises a reagent containing a nucleic acid molecule having a nucleotide sequence hybridizable with the DNA or a part thereof, or an RNA transcribed therefrom and detecting the DNA or the RNA.

A transgenic non-human vertebrate according to the present invention is a transgenic non-human vertebrate in which an expression of the DNA is modified or inducible the modification.

The animal is e.g. a peripheral nerve lesion model animal.

The knockout non-human vertebrate according to the present invention is the non-human vertebrate in which an expression of the intrinsic DNA is suppressed.

The knockout non-human vertebrate also includes a non-human vertebrate in which another gene is knocked out.

A cell according to the present invention includes a cell derived from any of the aforesaid non-human vertebrates.

A screening method according to the present invention also includes a method of screening a compound to increase or reduce an activity of an intrinsic promoter of the DNA shown in Figs. 1 to 4, comprising the steps of:
a) detecting an expression of a gene bound to a downstream region of the intrinsic promoter of the DNA shown in Figs. 1 to 4 under the presence of a compound to be tested; and
b) selecting the compound to increase or reduce the expression.

Moreover, the screening method of the present invention includes a method of screening a compound to increase or reduce an activity of an intrinsic promoter of the DNA shown in Figs. 1 to 4, comprises the steps of:
a) applying a compound to be tested to the non-human vertebrate or a cell derived from the animal; and
b) selecting the compound to increase or reduce an expression of a gene which is knocked in.

An animal immunized with a neurotonin according to the present invention is an animal immunized with the neurotonin which is to be used for studies of an Isaacs syndrome or other peripheral nerve lesions and for screening of a substance having an improvement action of the Isaacs syndrome or other peripheral nerve lesions.

According to the present invention, a rabbit is immunized with a neurotonin for.screening a protein or a non-protein substance which improves a suppressed VGKC function, a signal transduction disorder in a nerve terminal or a painful muscular convulsion.

The present invention provides a diagnosing marker for detecting a specific conjugation with the monoclonal antibody by using, as a marker, a neurotonin, a related protein thereto or other antibodies causing peripheral nerve lesions, thereby measuring a level of a neurotonin, a related protein thereto or an antibody causing the peripheral nerve lesions in a sample test body obtained from a subject to thereby diagnose an Isaacs syndrome or other peripheral nerve lesions.

The present invention provides an examination method of offering data for diagnosing an Isaacs syndrome or other peripheral nerve lesions, wherein a level of a neurotonin or an antibody causing peripheral nerve lesions in a sample obtained from a subject is measured.

The present invention provides a blood filtering material for use in eliminating a numbness caused by the presence of a neurotonin or an antibody causative peripheral nerve lesions to carry out a treatment which allows a neurotonin antibody or an antibody causing the peripheral nerve lesions in a blood to bind to the monoclonal antibody fixed onto the filtering material through a dialytic filtration of the blood, thereby reducing the neurotonin antibody or the antibody causative the peripheral nerve lesions in a body.

The present invention provides a vaccine composition for stimulating an immune response to an Isaacs syndrome or other peripheral nerve lesions in a human being or other animals, the composition serving to stimulate an immune response against at least one polypeptide described above which is contained together with a pharmaceutically acceptable carrier in the composition, or an immune response against a polypeptide encoded by a inserted nucleic acid molecule in a virus cell or the host cell which is contained in the composition.

The present invention provides a medium for a cell culture comprising the monoclonal antibody according to claim 31 or 32 for in vitro reproducing a cell to be a peripheral nerve by removing an antibody causative peripheral nerve lesions through an incubation with peripheral nerve and muscle system cells separated from a patient having peripheral nerve lesions, to thereby cause a specific binding of the causative antibody to the monoclonal antibody.

### Brief Description of the Drawings

Fig. 1 shows a nucleotide sequence of a human neurotonin coding cDNA (short).
Fig. 2 shows a nucleotide sequence of a human neurotonin coding cDNA (long).
Fig. 3 shows a nucleotide sequence of a mouse neurotonin coding cDNA (short).
Fig. 4 shows a nucleotide sequence of a mouse neurotonin coding cDNA (long).
Fig. 5 shows an amino acid sequence of a human neurotonin (short).
Fig. 6 shows an amino acid sequence of a human neurotonin (long).
Fig. 7 shows an amino acid sequence of a mouse neurotonin (short).
Fig. 8 shows an amino acid sequence of a mouse neurotonin (long).
Fig. 9 is a photograph showing the detection of a neurotonin and the estimation of its molecular weight by Western blotting, also illustrating the result of a rat (PC-12) together with an NB-1 nerve cell (left side). 66KDa corresponds to the neurotonin (long) and 44KDa corresponds to the neurotonin (short).
Fig. 10 is a photograph showing the induction of peripheral nerve lesions caused by immunizing a rabbit with a human neurotonin. A indicates a normal rabbit holding a natural lie-down position. B indicates a rabbit suffering from the peripheral nerve lesions induced after the immunization which has a numb hind limb and presents dysbasia.
Fig. 11 is a photograph showing the muscular tissue abnormality of a rabbit which is caused by an immunization with the neurotonin. A indicates the muscular tissue of a normal rabbit and B indicates the muscular tissue of the rabbit having the peripheral nerve lesions. A result obtained by an HE stain is shown in a 100-fold magnification.
Fig. 12 is a photograph showing a result obtained by detecting an antibody raised against a neurotonin in a human serum by using the human neurotonin. Horseraddish peroxidase conjugated - antihuman immunoglobulin M (anti - human IgM-HRP) was used as a secondary antibody. A indicates the serum of a patient M having the peripheral nerve lesions, B indicates the serum of a patient A having the peripheral nerve lesions, and C indicates the serum of a normal human.

### Detailed Description of the Invention

The present invention provides a peripheral nerve cell protein having the following properties,
wherein the protein
a) is found in peripheral nerve cells of Isaacs syndrome patients or other patients having peripheral nerve lesions,
b) reacts with an antibody found in a blood of the patients,
c) causes a similar symptom to an Isaacs syndrome group with an immunization to a rabbit, and
d) has a molecular weight of approximately 66 kDa according to SDS-PAGE.

The present invention will be described below in detail in order of a cDNA encoding a neurotonin, a related nucleic acid molecule, the neurotonin, the physiological function of the neurotonin, a related protein, a medical material and an application to animals.

In this specification, the "peripheral nerve lesions" indicate the disorders of peripheral nerve except an Isaacs syndrome. In the text, this term will be used in a broad sense including the Isaacs syndrome if necessary.

"VGKC" is an abbreviation of voltage gated potassium channel, which implies an electric-potential dependent potassium channel.

In "a neurotonin and a related protein thereto" and "a neurotonin or a related protein thereto", the "related protein thereto" also includes an antibody against neurotonin.

### cDNA Encoding Neurotonin

A cDNA encoding a neurotonin may be prepared by the application of an immunoscreening method using the serum of an Isaacs syndrome patient to a cDNA library corresponding to a human genome. More specifically, the cDNA library derived from a nerve such as an NB-1 cell is created by separating mRNAs through a conventional method and incorporating the mRNAs into a λ vector or the like by a well-known technique, and allowing a reverse transcriptase to act on the mRNA mixture. The serum containing the anti-neurotonin antibody is applied for the cDNA library so that a clone containing a cDNA expressing a neurotonin is identified

In a process in which the present inventor carried out the immunoscreening by using the sera from Isaacs syndrome patients to obtain a cDNA encoding a novel protein as described above, two types of cDNA having different chain lengths were found. The long cDNA encoding the protein will be referred to as a "long cDNA" while the short cDNA encoding a protein of 386 amino acids which was first obtained will be referred to as a "short cDNA".

Figs. 1 and 2 show a nucleotide sequence of the "short cDNA" and the "long cDNA" for the cDNA encoding the neurotonin.

The DNA according to the present invention encodes the neurotonin or includes the whole nucleotide sequence shown in Fig. 1 or 2. The DNA includes a DNA of a genome structural gene carrying genetic information before splicing as well as a DNA containing initiation and termination codons.

Furthermore, a nucleic acid molecule encoding the neurotonin, including at least one nucleotide sequence substantially corresponding to a whole or part of the nucleotide sequence shown in Fig. 1 or 2, or including a sequence which is substantially homologous with any of the sequences or hybridizable with any of the sequences also belongs to the nucleic acids of the present invention. The nucleic acid molecule according to the present invention may be a single or double stranded DNA, cDNA or RNA.

The "substantially homologous" sequence includes a sequence having approximately 50% or more, for example, 60% or more of a sequence identity, a sequence of functionally equivalent allelic variants, and a related sequence modified by the substitution, addition and/or deletion of at least one nucleotide. Similarly, "functionally equivalent" implies a similar correspondence to a polypeptide indicative of the same functionality as that of the neurotonin.

A nucleic acid molecule hybridizable with the sequences shown in Figs. 1 and 2, or the substantially homologous sequence or functionally equivalent sequence which is defined as described above is also included within the scope of the present invention. "Hybridize" used herein is defined as a sequence being bound under non-stringent condition (6 x SSC, 1% SDS, 10% Dextran, 100 g/ml salmon sperm DNA, a room temperature) and washed away under a low stringent condition (2 x SSC, a room temperature, more preferably 2 x SSC, 30°C) or a higher stringent condition, for example, 2 x SSC, 65°C. SSC is an abbreviation of Standard Saline Citrate and implies 0.15M NaCl and 0.015M sodium citrate pH 7.2.

By utilizing a well-known method of genetic engineering technology, it is also possible to create a nucleic acid molecule including at least one nucleotide sequence encoding a polypeptide of an antibody against the neurotonin and incorporating a region encoding at least one antigenic determinant from the neurotonin encoding sequence shown in Fig. 1 or 2. Such a nucleic acid molecule is also included in the present invention.

In order to detect a structural gene encoding the neurotonin and a DNA derived therefrom, a cDNA derived therefrom, or a DNA comprising at least one nucleotide sequence substantially corresponding to a whole or part of the nucleotide sequence shown in Fig. 1 or 2, a sequence which is substantially homologous with or functionally equivalent to any of the sequences described above, or a sequence hybridizable with any of the sequences and an RNA transcribed from the DNA, a reagent containing these nucleic acid molecules is preferably used based on a principle utilizing a hybridization with their DNA chain or RNA chain. Furthermore, these nucleic acid molecules can be also employed as hybridization probes for searching a PCR primer or a homologous sequence portion.

Conventionally, the probe can be labeled with a radioisotope, more preferably, a nonradioisotope such as a fluorescent dye or chemiluminescence by a 5' terminal label method, a nick translation method, a random primer method or the like. A reagent for detecting the DNA or the RNA transcribed therefrom is preferably utilized as a probe for a hybridization in an in situ hybridization, a southern hybridization, a northern hybridization, a plaque hybridization, a colony hybridization or the like.

Such a probe is also used preferably in searching for a target clone from a genome library or a cDNA library by the hybridization on a nucleic acid level.

### Neurotonin

The neurotonin according to the present invention is a protein having an amino acid sequence shown in Fig. 5 or 6, or a protein encoded by the cDNA, a gene corresponding to the cDNA or a DNA hybridizable with any of the nucleotide sequences described above. The neurotonin has such an activity as to be bound to an antibody found in the blood of the above-described patient, and furthermore, can be conjugated to a "14-3-3 protein" in a peripheral nerve cell.

The neurotonin may be obtained by immunoscreening utilizing the sera of an Isaacs syndrome patient or of other patients having peripheral nerve lesions. Alternatively, if the cDNA is available, the neurotonin can also be prepared through the transcription and translation of the cDNA by utilizing a well-known recombinant DNA technology.

Furthermore, the protein according to the present invention is extended to a polypeptide in such a chemical structure as to be a polypeptide having an amino acid sequence shown in Fig. 5 or 6, a polypeptide having an amino acid sequence in which at least one amino acid is deleted, substituted or added and/or inserted in the amino acid sequence shown in Fig. 5 or 6, a syntheticpolypeptide containing an amino acid sequence constituting the antigenic moiety of the neurotonin, a polypeptide of their variant polypeptides which is functionally equivalent or a polypeptide of a fused polypeptide type further containing their amino acid sequences, and these polypeptides are also included in the present invention. Moreover, the protein according to the present invention may be obtained by a partial or whole artificial synthesis or by modification of the protein obtained from a biological source in addition to the protein of the biological source. The protein of the biological source according to the present invention also includes a protein manufactured based on the recombinant DNA technology.

The "polypeptide" used herein includes both a protein having a full length sequence and a polypeptide having a shorter sequence than the protein. Consequently, an immunologically active domain of the neurotonin or a fragment having the domain is also included in the present invention. "Immunologically active" implies that the domain includes an epitope against an antibody directed to the protein and has an antigenicity. Accordingly, a fragment including such a domain retains the antigenicity. Similarly, the synthetic polypeptide comprising an amino acid sequence constituting the antigenic portion of the neurotonin is also included in the protein according to the present invention.

The term of "functionally equivalent" used above in relation to the amino acid sequence of the polypeptide is referred to the situation where an amino acid sequence is modified by the deletion, substitution, addition and/or insertion of at least one amino acid, or an amino acid sequence is otherwise obtained by chemically modifying the side group of an amino acid residue through phosphorylation or dephosphorylation, glucosylation, deglucosylation or the like, for example, and a substantially equivalent action or activity to that of the neurotonin is retained irrespective of the altered amino acid sequence. Such a functionally equivalent variant is generated as a natural biological mutation in some cases or can also be produced by utilizing a well-known technique. For example, a functionally equivalent recombinant polypeptide can be generated by using a well-known technique such as the mutagenesis at a specific site, the mutagenesis at an unspecific site, the enzymic cleavage and/or ligation of an amino acid or the like.

The protein, that is, the neurotonin and related proteins thereto can be used for analyzing an antibody to recognize and bind to the proteins. For this purpose, a reagent for an immunological analysis includes any of the proteins described above and can detect the antibody in a specimen through an antigen-antibody reaction and the measurement of detecting any entity linked to the reaction. As a detecting entity, it is possible to properly exploit well-known detecting means such as an immuno-antibody method, a method utilizing a radioactivity or the like in addition to various spectroscopic methods including fluorescence or chemiluminescence.

Since such a reagent for the immunological analysis serves to detect and analyze a specific complexing of the antibody to be a disease marker with the neurotonin or a related protein thereto, it can also be utilized for a diagnosis. More specifically, it is also possible to utilize the reagent for diagnosing the Isaacs syndrome or other peripheral nerve lesions or to assess a curative effect by measuring the level of the specific antibody against the neurotonin in a specimen from a subject or an antibody responsible for the peripheral nerve lesions.

As another embodiment, it is possible to use the reagent in order to detect a fused cell producing an antibody, that is, to efficiently detect a fused cell producing a specific target antibody from a large number of fused cells in the manufacture of a monoclonal antibody described hereinafter. For example, a protein such as the neurotonin to be an antigen is fixed onto a solid-phase support and thereby an antibody in a fused cell supernatant to be bound thereto is detected.

A substance capable of binding to the neurotonin or related proteins thereto or a compound having a ligand which can be bound thereto is useful for a substance to control the function of the neurotonin, including the antibody or a substance which can be utilized for an analysis. Furthermore, it is also possible to employ a compound having a suppressing function for the production of an anti-neurotonin antibody, a compound likely affecting the action of the anti-neurotonin antibody, or a compound capable of blocking the coupling'of the antibody to the neurotonin. These compounds may exhibit some pharmacological activity in the Isaacs syndrome or other peripheral nerve lesions.

Preferably, a screening method for selecting such substances includes the following steps:
a) a step of allowing the candidate compound of a ligand to come in contact with the neurotonin or related proteins thereto; and
b) a step of selecting a candidate compound having a binding activity to the protein.

For analyzing the protein, it can be measured through a specific binding to a ligand to be obtainable by the screening method using the protein as an antiligand.

Furthermore, it is preferable that a method of screening a compound inhibiting the binding of said protein to the ligand thereof should comprise the following steps:
a) a step of allowing the neurotonin or related proteins thereto to come in contact with the ligand under the presence of a candidate compound; and
b) a step of selecting a candidate compound having an activity to inhibit the binding of the protein to the ligand thereof.

The ligand or compound which can be obtained by the screening method may be an inhibitor for the neurotonin or related proteins thereto. By utilizing the binding of the neurotonin or related proteins thereto, accordingly, the compound obtained by the screening may be used as a medicine for a treatment of the peripheral nerve lesions, including the Isaacs syndrome.

### cDNA Encoding Mouse Neurotonin

It is possible to isolate a cDNA encoding a mouse neurotonin from a mouse cDNA library through hybridization cloning. For this purpose, it is also possible to use a full length sequence of the neurotonin cDNA as a probe, thereby screening a plaque containing a positive clone.

Figs. 3 and 4 show a nucleotide sequence of a cDNA encoding the neurotonin of a mouse which is thus isolated and sequenced.

A DNA according to the present invention includes a DNA encoding the mouse neurotonin or a DNA containing whole the nucleotide sequence shown in Fig. 3 or 4. Furthermore, the present invention also includes a nucleic acid molecule which encodes the mouse neurotonin, contains at least one nucleotide sequence substantially corresponding to a whole or part of the nucleotide sequence shown in Fig. 3 or 4, is substantially homologous with any of the sequences or contains a sequence hybridizable with any of the sequences. The nucleic acid according to the present invention may be a single or double stranded DNA, cDNA or RNA.

The "substantially homologous" sequence includes a sequence having approximately 50% or more, for example, 60% or more of a sequence identity, a functionally equivalent allelic variant, and a related sequence thereto modified by the substitution, addition and/or deletion of at least one nucleotide. "Functionally equivalent" implies to have a sequence encoding a polypeptide indicative of the same functionality as that of the neurotonin.

A nucleic acid molecule hybridizable with the sequences shown in Figs. 1 and 2, or the substantially homologous or functionally equivalent sequence as defined above is also included in the scope of the present invention. "Hybridize" used herein is defined as described above.

By utilizing a well-known genetic engineering technology, similarly, it is possible to create a nucleic acid molecule including at least one nucleotide sequence encoding a polypeptide of an antibody against the neurotonin and incorporating a region encoding at least one antigenic determinant from the neurotonin encoding sequence shown in Fig. 3 or 4.

The above-described matters, utilization and the like for the DNA encoding the human neurotonin or the nucleic acid molecule exactly apply to a nucleic acid encoding a mouse neurotonin and a related protein thereto.

### Mouse Neurotonin

A mouse neurotonin according to the present invention is a polypeptide having an amino acid sequence shown in Fig. 7 or 8. In addition to the mouse neurotonin, a polypeptide of the present invention, furthermore, is extended to a polypeptide in such a form as to be a polypeptide having an amino acid sequence in which at least one amino acid is deleted, substituted or added and/or inserted in the amino acid sequence shown in Fig. 7 or 8, a synthetic polypeptide containing an amino acid sequence constituting the antigenic portion of the neurotonin, their variant which is functionally equivalent or a fused polypeptide further containing their amino acid sequences. The "polypeptide" and "functionally equivalent" used herein are defined as described above.

The above-described matters, utilization and the like for the human neurotonin exactly apply to a mouse neurotonin and a related protein thereto.

### Physiological Function of Neurotonin and Neurotonin Gene

A human genome contains a structural gene having information about the amino acid sequence of a neurotonin which is a protein newly found by the inventor through an immunoscreening method utilizing a relevant antibody present in the serum of an Isaacs syndrome patient. The details of a position, an expression physiology and a control function thereof have not been known. Further elucidation of the problem as to whether the Isaacs syndrome or other peripheral nerve lesions result(s) from the excessive expression of the neurotonin or are(is) caused derivatively by an ectopic expression is awaited. Also the relationship between a neurotonin and an antibody thereto and the peripheral nerve lesions should be resolved.

A human neurotonin is also the protein with which immunization may evoke the symptom of the Isaacs syndrome in the animal other than a human being. It is possible to use any animal which gives an immune response, for example, Primates, sheep, rabbits, rats, mice or the like.

For example, in case of a rabbit, a fused protein with GST is produced by using the neurotonin containing 511 amino acid residues in a total length and the rabbit is immunized with the fused protein to be an antigen. After approximately six months since inoculation, it is observed that peripheral nerve lesions showing a similar symptom to the Isaacs syndrome are induced, for example, a numbness in the hind limbs. In the muscle of the rabbit immunized with the neurotonin, the size of muscular cells is nonuniform differently from a normal muscular tissue, and the denaturation of a nerve is also observed.

In the Isaacs syndrome patient, the expression of the neurotonin gene is enhanced so that the blood level of an antibody to the neurotonin encoded thereby is raised. The cause of the expression of the gene, the physiological function of the neurotonin, and the relationship with the disease condition of the Isaacs syndrome are unknown. Since an antibody production against the neurotonin is also increased, there are also caused likely peripheral nerve lesions based on the outcome of an auto-immunity disease. Together with these observations or the results of experiments, it is acknowledged that the newly found and identified neurotonin is a protein closely associated with the Isaacs syndrome or other peripheral nerve lesions.

The cDNA of a mouse neurotonin is homologous by 85% or more with the nucleotide sequence of a human cDNA corresponding thereto. In an amino acid sequence level derived from the mouse neurotonin cDNA, another factor on homology is not found and its function is also unknown. The mouse neurotonin gene also functions in the same manner as the human neurotonin gene. It can be supposed that similar peripheral nerve lesions to the Isaacs syndrome are induced, for example, a hind limb has a numbness if the mouse is immunized with the human neurotonin.

Referring to the expression of the gene, a compound to increase or reduce the activity of the intrinsic promoter of the DNA may be screened by a method comprising the steps of:
a) detecting the expression of a gene bound to the downstream region of the intrinsic promoter of the DNA under the presence of a compound to be tested; and
b) selecting the compound to increase or reduce the expression.

The nucleotide sequence of the human neurotonin cDNA is converted into a corresponding amino acid sequence to carry out a motif search. Consequently, it is clear that a main motif is absent but a site to be modified by a lipid is present on the C terminal side. Based on the fact that the hind limb of the rabbit had a numbness through the immunization using the portion on the C terminal side of the neurotonin and that more antibodies to the neurotonin existing in the serum of a patient complaining of the numbness are found to be those for the C terminal region of the neurotonin, the neurotonin is a membrane protein and at least the C terminal portion thereof functions in a membrane.

From the present inventor's study, it has been elucidated that the C terminal portion of the neurotonin is bound to a "14-3-3 protein". The "14-3-3 protein" is present in a cell and can recognize proteins having phosphorylated serins residues, and its role as various modulators for a signal transduction is assumed. Accordingly, a protein or a nonprotein substance capable of binding to or interacting with the "14-3-3 protein" in place of the neurotonin or competitively may exert a similar influence to the neurotonin on the "14-3-3 protein". It is likely that such a protein or nonprotein substance may exhibit some pharmacological action in the Isaacs syndrome or other peripheral nerve lesions.

It has been reported that an antibody to VGKC is detected in the serum from some of patients having the Isaacs syndrome or other peripheral nerve lesions. For these disease conditions, the functional abnormality of VGKC is suggested to be responsible. Furthermore, it is apparent from the study of the present inventor that various antibodies against the neurotonin are also present in the sera of the patients, and particularly, a large number of patients have the antibody against the C terminal region of the neurotonin. Similarly, these antibodies are also assumed to be involved in the symptom in relation to the physiological function of the neurotonin. For this reason, an antibody which can bind to these antibodies, a compound capable of exerting a suppressing action on the antibody production, a compound which might influence the action of the antibody, a compound capable of blocking the binding of the antibody to the neurotonin or the like may exhibit some pharmacological action in the Isaacs syndrome or other peripheral nerve lesions.

### Related Nucleic Acid Molecule

An antisense RNA and an antisense DNA incorporating the neurotonin gene, an mRNA, a cDNA or a nucleic acid molecule related thereto according to the present invention by a recombinant DNA technology as well as, a ribozyme, a cosmid and the like are also included in the scope of the present invention. If necessary, the recombinant DNA and the nucleic acid molecule can also be utilized in various gene cloning operations applicable in the establishment of gene diagnosing methods and gene therapy and the development of drugs.

### Antisense RNA and antisense DNA

Since a double stranded RNA or DNA produced by a hybridization of an mRNA chain carrying the translation information of the neurotonin with an antisense chain complementary thereto, or an antisense RNA or antisense DNA against the cDNA or the nucleic acid molecules above described has an ability of inhibiting a translation through a hybridization with the complementary mRNA thereto and suppressing thereby the expression of a corresponding gene, it may be applied to an antisense medical treatment and the creation of a knockout mouse.

In the antisense medical therapy, the antisense RNA and the antisense DNA are utilized in the clarification of the mechanism of a disease onset on a gene level, the development of a treating method and the like. In order to control the expression of the neurotonin gene, moreover, it is possible to utilize the RNA and the DNA. More particularly in order to suppress the expression of a target gene, the antisense RNA may be injected into a cell, or alternatively the DNA to create the antisense RNA may be introduced therein.

### Ribozyme

The present invention also includes, among the ribozymes whose enzymatic activity has been demonstrated, the ribozyme designed to specifically cleave an optional nucleotide sequence of the RNA for the neurotonin, a related RNA transcribed from the above-mentioned cDNA or the nucleic acid molecule, or a related RNA transcribed from a part thereof and produced by a chemical synthesis, or an oligo DNA having the nucleotide sequence of the enzyme active site and an RNA-cleaving activity.

### Cosmid

Since a circular DNA incorporating the neurotonin gene, a DNA fragment thereof or a related nucleic acid thereto as well as necessary elements, such as, an origin of replication derived from a plasmid, a cos site of a λ bacteriophage are replicable in the same manner as the plasmid, it can be utilized as a vector. Such an artificial nucleic acid particle may incorporate therein a comparatively large DNA fragment. Therefore, for the purpose of cloning, it is possible to efficiently introduce the artificial nucleic acid particle into a bacterium such as Escherichia coli. Moreover, the artificial nucleic acid particle is also suitable for the preparation of a gene library.

### Expression Vector and Cloning Vector

A microorganism cell such as a prokaryotic cell or a eukaryotic cell which contains the transformed nucleic acid may be used for the expression in a large amount of a protein of interest.

The nucleotide sequence encoding the neurotonin according to the present invention can be so cloned as to bring about its expression by properly utilizing a group of well-known techniques and expression systems, for example, an expression system in the prokaryotic cell such as Escherichia coli or a Bacillus subtilis, an expression system in the eukaryotic cell such as a yeast or a transformed mammalian cell, an expression system in a transgenic mammals and the like.

It is possible to preferably use a method of preparing the synthetic polypeptide related to the neurotonin, comprising the steps of culturing the eukaryotic cell or prokaryotic cell incorporating the nucleic acid molecule under such a condition that the polypeptide is expressed, and collecting the polypeptide thus produced.

As described above, the scope of the present invention encompasses a cloning vector and an expression vector which contain the nucleic acid molecule or the nucleotide sequence according to the present invention. Such an expression vector incorporates a proper control sequence in which a reading frame and a nucleic acid molecule according to the present invention are fittingly ligated, for example, a translation control element such as start and stop codons, and transcriptional control elements such as a promoter operator region, ribosomal binding sites and an ending stop sequence.

Examples of the vector according to the present invention include plasmid, bacteriophage, a virus (including a eukaryotic virus) and the like which have been well-known in the recombinant DNA technology field or described in the documents in the art. These vectors may be used in various well-known expression systems. As a preferred viral vector, a baculovirus, an adenovirus, a vaccinia virus and the like can be illustrated.

In order to include an expression of the nucleic acid molecule or the nucleotide sequence according to the present invention by using various well-known techniques, the vector or cosmid may be inserted into a prokaryotic cell or a eukaryotic cell. Alternatively, it is possible to prepare a transformant holding the vector or cosmid in a germ cell line or a somatic cell in order to create a transgenic animal.

Furthermore, the present invention also includes a eukaryotic or prokaryotic host cell or a transgenic microorganism which has the nucleic acid molecule or polynucleotide molecule according to the present invention and is transformed or transfected. In order to produce these transformants and the like, a well-known transforming technique or transfection technique may be used.

If the neurotonin requires a post-translational modification such as a disulfide bonding, glycosylation or a lipid attachment more or less, a mammal host cell can well reproduce the native morphology of an antigen and an epitope. Therefore, the mammal cell expression system is more preferable. More specifically, the eukaryotic expression system can perform the post-translational modification whereas the Escherichia coli poorly reproduces the native morphology of the antigen and might produce an insoluble protein. Examples of an animal cell which may be preferably used for this purpose include a human or animal fibroblast or a myeloma cell line, for example, an Hela-human cell line, a BHK-infant hamster renal cell, a VERO monkey renal cell line, an FR3T3-Fisher rat fibroblast, an NIH3T3 - mouse fibroblast cell line, a C127I - mouse breast cancer cell line, a CV-1-Africa green monkey renal fibroblast, a 3T6 - mouse embryofibroblast, an L cell - mouse cell line, a CHO - Chinese hamster ovary cell line, an NSONSI - SP2 and other mouse myeloma cell lines, YB2/0 and Y3 mouse myeloma cell lines, rat myeloma cell lines and the like.

Various proper vectors to the mammal cell lines of different classes have been known and generally include a promoter and/or an enhancer which are (is) operatively ligated to a nucleotide sequence encoding a neurotonin or a fragment thereof. Examples of such a suitable promoter include an SV40 initial or late promoter, for example, a PSVL vector, a cytomegalovirus (CMV) promoter, a mouse metallothionine I promoter, a mouse breast cancer virus LTR (long terminal repeat) and the like. The vector carries a suitable marker, e.g., a gene therefor such as a gene of dihydrofolic acid reductase or a glutamine synthetase.

The transfection of a host cell can be carried out by using a standard well-known technology. For this purpose, for example, there has been established a calcium phosphate method, a method using DEAE-dextran or polybrene in place of the calcium phosphate, a protoplast fusion method, an erythrocyte ghost fusion method, a liposome fusion method or a lipofection method, a direct microinjection method, and a transfection method for mammal cell lines using a gene cannon or an electroporation. In general, a linear DNA may be introduced more easily than a circular DNA.

### Related Protein

A synthetic polypeptide related to a neurotonin may be utilized in an antibody production, a vaccine manufacture and the like which will be described below in the same manner as the neurotonin. The synthetic polypeptide can be produced by an expression in a host cell containing a recombinant DNA which is operatively ligated to an expression control sequence and includes the above-described nucleotide sequence, or in a host cell containing a vehicle or a vector carrying the recombinant DNA molecule. Alternatively, the polypeptide may also be expressed by directly injecting a bare DNA molecule of the present invention into the host cell.

The thus expressed synthetic polypeptide may be a fused polypeptide containing a portion exhibiting an immunogenicity of a whole or part of a neurotonin or a further additionally fused polypeptide encoded by the DNA of a recombinant molecule fused thereto. For example, it is desirable to prepare the synthetic neurotonin according to the present invention which is coupled to a protein such as glutathione-S-transferase, phosphatase, β-galactosidase, urease or an HB core (hepatitis B core) antigen or a fused protein thereof containing another polypeptide. Most of such fused proteins are formed by the expression of a recombinant gene in which two coding sequences are bound to a tuned reading frame. Alternatively, the polypeptide can be combined by chemical means in vitro. Such a fused or hybrid derivative is also included in the present invention and the synthetic polypeptide can be manufactured by using chemical methods such as a well-known Merrifield solid-phase synthesizing method.

### Antibody Protein

Antibodies to a neurotonin and a related protein thereto are produced in an Isaacs syndrome patient and a patient having other peripheral nerve lesions. Accordingly, various antibodies to the neurotonin are also found to be present in the sera of the patients. Particularly, in the studies of the present inventor, an antibody directed to the C terminal side portion was observed in many patients. These antibodies are also likely involved in a symptom in close relation to the physiological function of the neurotonin. Consequently, an antibody which can be bound to these antineurotonin antibodies may also achieve some pharmacological function in the Isaacs syndrome or the peripheral nerve lesions.

The antibody may be prepared by inoculating animals with a protein to be an antigen and immunizing the animals. Examples of the animals for this purpose include rabbits, sheep, mice, rats and the like which have widely been utilized. However, all the antibodies to be raised are polyclonal which are antibodies resulting from the nonhuman animals, and therefore, are foreign substances for a human.

Among the antibodies, a monoclonal antibody is a uniform antibody capable of specifically binding to a single epitope which is particularly useful and preferable. More specifically, the scope of the present invention includes a monoclonal antibody to a neurotonin, a monoclonal antibody to a VGKC antibody, a monoclonal antibody to an antibody causing the peripheral nerve lesions or other related proteins and the like.

In the preparation of these monoclonal antibodies, it is also possible to utilize a conventional established cell fusing technique for preparing and cloning a hybridoma of a lymphocyte cell obtained from an animal spleen immunized with the neurotonin to be an antigen and a myeloma cell having an HAT sensitivity or to utilize a well-known recombinant DNA technology, resulting in a mass production.

In order to obtain the humanized monoclonal antibody, a chimera or a humanized antibody is produced by the recombinant DNA technology including a fusion of a human antibody cDNA and an antibody cDNA derived from an animal cell obtained from a mouse, a rat or the like. It is also possible to employ a method of producing a human antibody by the utilization of a human derived cell and tumor cell clone, the fusion of a mouse tumor cell and a human cell and the utilization of a transgenic mouse.

Examples of the general application of the antibody include testing chemicals, diagnosing chemicals, a remedy, a reagent for a study, a reagent for an assay, an antibody for immunoscreening and the like, related to a neurotonin, a related protein thereto, and a "14-3-3 protein". Examples of the utilization embodiment of these antibodies include a reagent for an immunological assay to measure the neurotonin or the related protein thereto which includes an antibody to react with the neurotonin or the related protein thereto or particularly is based on the fact that the antibody is detectably bound to the neurotonin or the like. The "detectable" implies herein that well-known detecting means such as an immune antibody method or a method utilizing a radioactivity as well as various spectroscopic methods may be appropriately applied. Examples of a specific utilization manner include a radioimmunoassay (RIA), an enzyme-labeled antibody measuring method (ELISA) and the like. In the RIA, the antibody labeled by ¹²⁵I or ¹³¹I or the like is fixed to a solid-phase support and a radioactivity thereof is measured. In the ELISA, moreover, the antibody is fixed to the support in the same manner and a secondary antibody attaching alkaliphosphatase, horseradish peroxidase or the like is added to carry out an enzyme reaction, thereby resultingly a color or fluorescence is measured.

As another example of such an embodiment as to utilize the antibody, moreover, there can be proposed a test method of diagnosing the Isaacs syndrome or other peripheral nerve lesions and offering data for assessing the effect of a treatment. This test method is characterized in that a neurotonin or related protein thereto in a sample obtained from a subject is examined, or the level of a neurotonin antibody or an antibody causing the peripheral nerve lesions is measured.

Furthermore, a specific binding with the above-described antibody is detected by using, as a marker, the neurotonin or related protein thereto, or the antibody causing the peripheral nerve lesions. Thus, the level of the neurotonin or related protein thereto in the specimen obtained from the subject, or the level of the antibody causing the peripheral nerve lesions is measured. An obtained result is offered to diagnose the Isaacs syndrome or other peripheral nerve lesions or to assess the treatment effect. Referring to these reagents for an analysis, the neurotonin, the related protein thereto or the like to be analyzed is originally present in a nerve cell. By analyzing the antibody present in the body fluid of the subject, however, it is possible to detect the neurotonin or related protein thereto.

By setting, as a clue, the expression of a gene encoding the neurotonin or related protein thereto, it is possible to detect or separate a cell from which said protein is produced. By using an antibody against the neurotonin or related protein thereto, preferably a monoclonal type antibody thereto as detecting means to utilize a flow cytometry method, for example, it is possible to separate or detect a cell expressing these proteins. As a reagent for the detection or the separation, the antibody, more preferably the monoclonal antibody includes an antibody obtained by attaching a fluorescent substance as a label, for example. More specifically, the detection is carried out by following the fluorescent excitation of the antibody bound to the cell and the separation is carried out by means of a cell sorter having an automatic sorting function.

By using this method, it is possible to specifically detect or separate the peripheral nerve cells expressing the neurotonin in a cell population. By using this method, for example, it is possible to detect and separate peripheral nerve cells derived from a transgenic non-human vertebrate or a knockout non-human vertebrate which will be described below. These cells provide a useful material for examining the expression and control of a neurotonin gene.

The present invention also includes a eukaryotic or prokaryotic host cell which comprises the nucleic acid molecule or the nucleotide molecule according to the present invention and is transformed or transfected or a transgenic microorganism. Furthermore, the present invention also includes a fused cell of a B cell producing the monoclonal antibody and a tumor cell. The antibody and the flow cytometry is combined and utilized, and can also be utilized for a separation of the transformant, the fused cell or the like of objects.

### Application as Medical Material

The neurotonin and related protein thereto according to the present invention may be utilized for the analysis, the clinical test, the diagnosis and the treatment, and furthermore, can be applied to various medical materials, for example, a blood filtering material, a cell culture medium, a carrier of a drug delivery system, a vaccine and the like. While the utilization embodiments will be demonstrated as an example, the present inventions are not restricted to them.

If an antineurotonin-antibody or an antibody causing the peripheral nerve lesions in a blood is bound to the above-mentioned monoclonal antibody fixed to a filtering material, and through a dialytic filtration of the blood, the filtered blood is then returned to a patient again, it is possible to reduce the neurotonin antibody or the antibody causing the peripheral nerve lesions in a body. Such filtration of the blood in exchange for a whole blood dialysis provides a therapeutic method of releasing a numbness caused by the neurotonin or the antibody thereof, or the antibody causing the peripheral nerve lesions. The present invention also provides the utilization of a blood filtering material binding the antibody for this purpose.

As another embodiment, there can also be proposed the use of a cell culture medium comprising a monoclonal antibody reacting specifically with the neuroton in through an incubation with peripheral nerve and muscle system cells separated from a patient having the peripheral nerve lesions to bring about binding to the antibody concerned in the cause of the peripheral nerve lesions to thereby remove the antibody, for in vitro reproducing a cell to be a peripheral nerve.

A humanized antibody has no antigenicity to a human. Therefore, there can be proposed the utilization of such humanized antibody to be means for a treatment through binding directly to the neurotonin or other proteins, biological components and the like which are concerned in the cause of the peripheral nerve lesions including the Isaacs syndrome to thereby lose their function, or to be a DDS (Drug Delivery System) carrier to effectively transport an effective bioactive substance or a compound having the drug efficacy to a target site. In view of reaction uniformity, the human monoclonal antibody may be used particularly suitably.

A vaccine composition according to the present invention stimulates an immune response to the Isaacs syndrome or other peripheral nerve lesions in a human or other non-human animals. The vaccine may contain any polypeptide of the proteins belonging to the scope of the present invention, together with a pharmaceutically-acceptable carrier to thereby stimulate an immune response to the polypeptide, or contain a virus cell or a host cell having any of the above-mentioned nucleic acid molecules inserted therein to thereby stimulate an immune response to a polypeptide to be encoded by the inserted nucleic acid molecule.

The vaccine composition can be manufactured according to a well-known method in a vaccine manufacturing field. Such a vaccine prescription may conventionally include at least one polypeptide according to the present invention together with at least one appropriate adjuvant, for example, aluminum hydroxide, saponin, Quil A or a refined form thereof, muramyldipeptide, mineral oil, novasom or the like if it is proper under the presence of at least one pharmaceutically acceptable carrier or diluent. Examples of a preferable carrier include a liquid medium such as a physiological saline solution suitable for a vehicle to be used for introducing a peptide or a polypeptide to a patient. An additive component such as an antiseptic may be contained.

As another vaccine prescription, it may also contain a virus in which the nucleic acid molecule according to the present invention is inserted, or a host cell, or a microorganism such as a vaccinia virus, an adenovirus or a Salmonella tribe. This is for stimulation of an immune response to the polypeptide to be encoded by the inserted nucleic acid molecule.

Accordingly, another aspect of the present invention is the method using the nucleic acid molecule or polypeptide according to the present invention in the manufacture of the vaccine composition to stimulate the immune response to the Isaacs syndrome or the other peripheral nerve lesions in the human or the other animals.

For the administration of such a vaccine, a conventional route, for example, an oral or non-oral administration can be optionally utilized (for example, an intramuscular injection at an optional interval, that is, two injections every 7 to 28 days).

### Animal

In order to search the neurotonin gene and the function and expression control of the neurotonin and to establish a method of diagnosing and treating peripheral nerve lesions including the Isaacs syndrome based thereon to develop a remedy therefor, a disease model animal is useful. More specifically, an animal model for peripheral nerve lesion according to the present invention is a transgenic non-human vertebrate which could be modified in the expression of a DNA encoding the neurotonin or a DNA which is homologous to or hybridized with a nucleotide sequence shown in Fig. 1 or 2 or a nucleotide sequence shown in Fig. 3 or 4, or could induce the modification.

Examples of a more useful transgenic non-human animal include a non-human vertebrate, that is, a knockout non-human vertebrate in which the expression of an inherent neurotonin gene DNA or a related DNA thereto is suppressed and a non-human vertebrate having other genes knocked out. Such other genes exclude the gene encoding the neurotonin and may be genes encoding other proteins of a peripheral nerve cell, for example, a "14-3-3" protein or the like. The type of the non-human vertebrate is not restricted. These animals can also be utilized as the model animals of the peripheral nerve lesions for the above-mentioned purpose.

As the non-human vertebrate to be used particularly preferably, it is possible to easily produce a transgenic mouse having the gene knocked out. Such a mouse can be widely utilized for the elucidation of the mechanism of the peripheral nerve lesions, in addition to the function of the gene which is knocked out, the establishment of the diagnosing and treating method, the development of chemicals, the test of a vaccine and the like. Such a transgenic mouse can be created by a well-known method of forming, in a mouse fertilized ovum, a DNA reversely transcribed to the gene to be knocked out or by a well-known antisense method of introducing an antisense RNA. In these cases, such production can be carried out through a targeting vector using a neurotonin gene obtained from a mouse genome. Furthermore, it is also possible to use embryonic stem cells, that is, ES cells.

An animal immunized with the neurotonin may be used for screening a compound having the improvement action of the Isaacs syndrome or the other peripheral nerve lesions. Such an animal is not particularly restricted to but may be of any type evoking an immune response. For this purpose, the animals such as rabbits, sheep, mice and rats have widely been utilized.

For example, in the rabbit immunized with the human neurotonin as described above, an antibody against the human neurotonin is raised before and after six weeks and a similar symptom to the Isaacs syndrome is manifested. The rabbit having the similar symptom to the Isaacs syndrome may be used for examining the function of antibodies such as the monoclonal antibody or the effectiveness of vaccines and chemicals or screening a compound blocking a factor to be bound to the neurotonin, a compound bound to the protein to modulate the function thereof, a protein or a non-protein substance which improves the suppression of a VGKC function, a signal transduction disorder in a nerve terminal or a painful muscular convulsion, or the like.

A compound to increase or reduce the activity of an intrinsic promoter of the DNA in relation to the expression of the neurotonin gene can be screened by a method other than the method described in the column of "related protein", the method comprising the steps of:
a) applying a compound to be tested to a non-human vertebrate or a cell derived from the non-human vertebrate, and b) selecting the compound to increase or reduce the expression of the gene which is knocked in.

### Advantage of the Invention

According to the present invention, the neurotonin supposed to be the key substance of the Isaacs syndrome has been identified and the structure thereof was determined. Therefore, it is possible to produce or prepare a monoclonal antibody thereto, a compound capable of modulating the function of the substance, an animal applicable to screening the compound and the like. It is possible to establish a testing method and a diagnosing and therapeutic method for the peripheral nerve lesions including the Isaacs syndrome by utilizing the neurotonin, the related substance thereto and the animals.

According to the present invention, the presence of the neurotonin gene is demonstrated by the isolation and identification of a cDNA thereof. By utilizing an antibody to the neurotonin or to the antibody causing the peripheral nerve lesions, a monoclonal antibody and a transgenic mouse, consequently, useful means for studies of the function of the neurotonin gene and a mechanism of the expression, a regulating function and the like are provided, and furthermore, clarification of the mechanism of a functional disorder in the peripheral nerves, an entity and the like can be carried out.

### Example

The present invention will further be described below based on examples and the scope of the present invention is not restricted to the description.

### Example 1

Gene cloning of an antigen (neurotonin) recognized by the serum of an Isaacs syndrome patient

A cDNA encoding a neurotonin was obtained by immunoscreening using the serum antibody of an Isaacs syndrome patient expressing the corresponding gene of a human genome. Total RNAs were extracted from human neuroblastoma (NB-1) strains by an acid guanidine/phenol chloroform method, and furthermore, mRNAs were separated by an affinity chromatography using oligo (dT) beads (Anal. Biochem., 162:159, 1987). By using a λ ZAP vector (STRATAGENE Co., Ltd.), a cDNA library was formed in accordance with an ordinary method. By using a picoBlue immunoscreening Kit (STRATAGENE Co., Ltd.), an immunoscreening using the serum of the patient was carried out. A positive clone (phage) thus obtained was converted into a plasmid pBluescript II SK(+) by a helper phage. The nucleotide sequence of a DNA inserted in the pBluescript II SK(+) was determined by an ABI PRISM377 DNA Sequencing System (Perkin Elmer Co., Ltd.) based on a dye terminator method (Proc. Natl. Acad. Sci. USA., 74:5463, 1977) using M13PrimerM4 and M13PrimerRV(Takara). A nucleotide sequence was determined from the 3' terminal of the cDNA encoding an antigen (neurotonin) recognized by the serum of the Isaacs syndrome patient to elucidate a nucleotide sequence containing a Poly(A)⁺ chain (Fig. 1). The nucleotide sequence was subjected to a homology search with GenBank. As a result, a similar sequence has not been reported and it was found that the nucleotide sequence is a novel gene. The cDNA (referred to as a "short cDNA") is constituted by 1347 nucleotides in total and encodes a novel protein comprising 386 amino acids.

Although an attempt for obtaining a protein to be encoded by the cDNA was made, the methionine initiation codon was not found. For this reason, the nucleotide sequence was used to perform PCR by a 5'-RACE (Rapid Amplification of cDNA Ends) method(Proc. Natl. Acad. Sci. USA., 85:8998-9002,1988) through the cDNA library. Consequently, a cDNA (referred to as a "long cDNA") corresponding to a protein consisting of 511 amino acids in total, that is, the neurotonin was obtained.

Figs. 1 and 2 show "short cDNA" and "long cDNA" nucleotide sequences, respectively.

### Example 2

An expression of a recombinant protein in Escherichia coli

A short cDNA encoding a part of a neurotonin was extracted from a cDNA clone obtained by the immunoscreening according to the example 1. The short cDNA having a recognition sequence for an EcoRI/XhoI at a terminal was inserted into a glutathione S-transferase (GST) fused protein expression vector pGEX-5X-3 to perform subcloning. The pGEX-5X-3 incorporating the short cDNA was introduced into a BL21 Escherichia coli strain by a heat shock method at 42°C for 45 seconds so that a BL21/short cDNA-GST gene/pGEX-5X-3 was obtained. The BL21 was cultured in an LB culture medium containing 0.1mg/mL ampicillin, and 0.1mM isopropylthio-β-D-galactoside (IPTG) was added, and furthermore, the cultivation was carried out at 37°C for two hours to induce the expression of the aforesaid fused protein. The BL21 collected by a centrifugation was washed with PBS (Phosphate buffered saline) and was then subjected to 1mg/mL lysozyme digestion, and was solubilized with 0.1% Triton X-100. A BL21 derived protein suspension containing the solubilized GST fused protein was applied to a glutathione Sepharose 4B (GS4B) column and was then washed with PBS to purify a target GST-short neurotonin fused protein by 50mM reduced form glutathione/PBS. Furthermore, a molecular weight, a homogeneity, a subunit structure and the like were examined by SDS-electrophoresis (SDS-PAGE).

An expression of a protein by a full length cDNA recombinant encoding a neurotonin in Escherichia coli

A neurotonin cDNA was obtained by adding two molecules of an influenza hemagglutinin (HA)-tag to the 3' terminal of a full length cDNA having the EcoRI/XhoI recognition sequence at a terminal which was obtained in the Example 1, was inserted into a glutathione S-transferase (GST) fused protein expression vector pGEX-5X-1 as an expression vector and was subjected to subcloning. The pGEX-5X-1 incorporating the cDNA was introduced into a BL21 Escherichia coli strain by a heat shock method at 42°C for 45 seconds so that a BL21/long cDNA-GST gene/pGEX-5X-1 was obtained. The BL21 was cultured in an LB culture medium containing 0.1 mg/mL ampicillin and 0.1mM isopropylthio-β-D-galactoside- (IPTG) was added. Furthermore, the cultivation was carried out at 30°C for three hours to induce the expression of a fused protein (GST-neurotonin-HAHA) attaching GST and HA to N and C terminals respectively. The BL21 collected by a centrifugation was washed with PBS and was then subjected to 1mg/mL lysozyme digestion, and was solubilized with 0.1% Triton X-100. A BL21 derived protein suspension containing the solubilized GST fused protein was applied to a glutathione sepharose 4B (GS4B) column and was then washed with PBS to purify a target GST neurotonin-HAHA fused protein by 50mM reduced form glutathione/Tris-HCl (pH8.0).

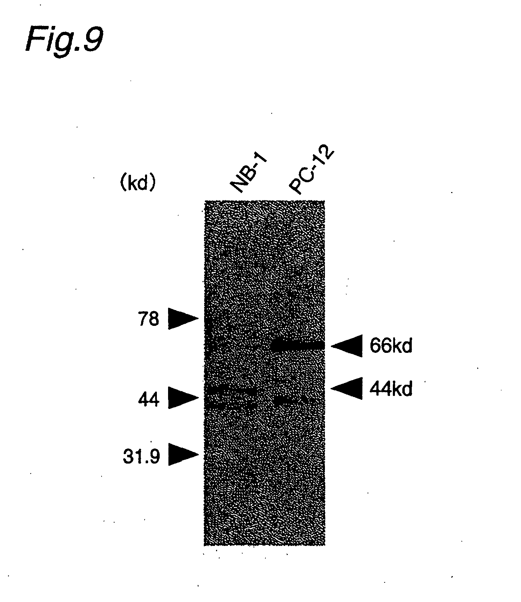

For the confirmation of the expression, a fraction eluted with 50mM reduced form glutathione was diluted in 1/200 and 1/2, 000 with PBS and was then treated with 25mM Tris-HCl (pH6.8), containing 0.25% SDS, 0.05% mercaptoethanol and 0.1% glycerol, and was thereafter applied to 8% SDS-PAGE. After the SDS-PAGE, the fused protein (GST-neurotonin-HAHA) was transferred to a nylon membrane by an electroblotting method. The nylon membrane was subjected to blocking at a room temperature for 60 minutes with PBS containing 5% skimmed milk and then an immunoreaction was effected at a room temperature for 60 minutes with an anti-HA monoclonal antibody (Boehringer Mannheim Co., Ltd.) diluted in 1/400 with PBS containing 0.5% skimmed milk. After the reaction, washing was conducted with 0.1% Tween 20/PBS and another immunoreaction was carried out at a room temperature for 60 minutes by using a horseradish peroxidase conjugated -antimouse-immunoglobulin G (anti-mouce IgG-HRP) as a secondary antibody, and washing was then conducted with 0.1% Tween 20/PBS to detect a target antigen through manifesting an HRP activity. ECL (Amersham Co., Ltd.) was used for the detection of the HRP activity (Clin. Chem., 25:1531, 1979). A result is shown in Fig. 9. The molecular weight of the neurotonin was estimated to be approximately 66KDa based on the molecular weight size of the GST-neurotonin-HAHA fused protein.

An expression of a protein in vitro by a full length cDNA recombinant

A neurotonin cDNA (Fig. 2) was modified at its terminal by a restriction enzyme EcoRI and was then inserted in a pBluescript IIKS vector. Thereafter, by using the pBluescript (1µg) and TNT-coupled Translation System (Promega Co., Ltd.), a neurotonin was expressed as a (³⁵S) labeled entity in vitro by an in-vitro translation method. The (³⁵S) labeled protein was applied to 10% SDS-PAGE and a radioactivity was detected by an imaging analyzer (BAS2000, Fujix). It was observed that the molecular weight of the neurotonin translated via the neurotonin cDNA in vitro based on the SDS-PAGE is approximately 66KDa.

### Example 3

A confirmation of an expression of a neurotonin gene by a northern blotting method

mRNAs were isolated froman NB-1 cell, an A549 cell strain, a Jurkat cell strain and an HeLa cell strain by the conventional method. 1µg of the mRNAs was separated by 1% agarose gel electrophoresis and was transferred to a nylon membrane by a contact blotting method. The nylon membrane was treated at 80°C for two hours and then a prehybridization was carried out at 42°C for two hours in a Denhardt's solution. Next, a neurotonin cDNA using a neurotonin cDNA radioactive-labeled with ³²P as a probe was hybridized with the mRNA at 42°C for 12 hours. The nylon membrane thus obtained after the reaction was washed with 300mM NaCl and 30mM sodium citrate and was then washed again at 50°C by using 15mM NaCl and 1.5mM sodium citrate. A target mRNA was detected by photosensitizing an X-ray film. From an autoradiograph thus obtained, it was observed that a neurotonin gene was strongly expressed in the NB-1 cell of a nerve cell.

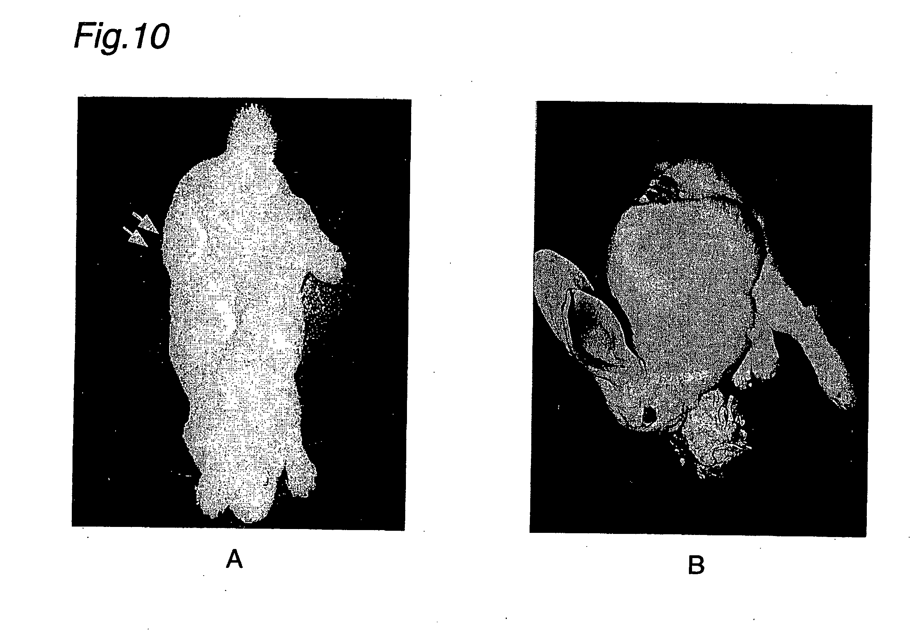

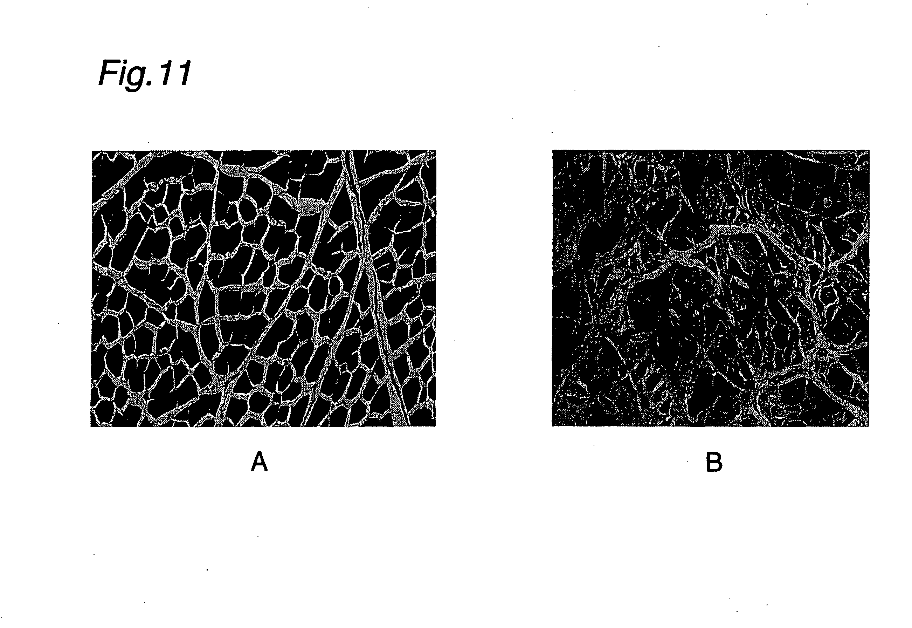

An immunizationa of a rabbit with a neurotonin (Figs. 10 and 11)

By using a neurotonin comprising 511 amino acids in total, a fused protein thereof with GST was produced and was then used as an antigen to immunize three rabbits together with an adjuvant in accordance with an ordinary method. After six weeks, it was observed that peripheral nerve lesions having similar symptoms to the Isaacs syndrome, for example, a numbness on hind limbs, a muscle rigidity, dysbasia based thereon and the like were induced to all the three rabbits (Fig. 10). A muscle obtained from the rabbit immunized with the neurotonin was subjected to HE staining and was observed microscopically. As compared with a normal muscular tissue, consequently, the size of a muscular cell was nonuniform and the denaturation of a nerve cell was also observed (Fig. 11).

Next, a human neurotonin was divided into three sections, that is, a C terminal region portion, an N terminal region portion and their middle portion. They were separately immunized to five rabbits. Consequently, the above-mentioned symptoms were induced to the rabbit immunized to the C terminal portion of the neurotonin. In the case in which other portions were immunized, such a result was not obtained in the rabbits.

A serum obtained from the rabbit immunized with the neurotonin was used as an anti-neurotonin antiserum in the following experiments.

A confirmation of an expression of a neurotonin gene in various cells by a Western blotting method

By using a GST-short neurotonin fused protein (positive control) and an NB-1 nerve cell which were prepared in the Example 1 and an HEK (human embryonic kidney) - 293T, the expression state of a neurotonin gene was confirmed by a Western blotting method.

First of all, cytolysates solubilized with 1% NP-40 were prepared from various cells to be samples. Each of the cytolysates was treated with 25mM Tris-HCl (pH6.8), containing 0.25% SDS, 0.05% mercaptoethanol and 0.1% glycerol and was separated through 8% SDS-PAGE. After the SDS-PAGE, proteins derived from various cells were transferred to a nitrocellulose (NC) membrane by an electroblotting method. On the NC membrane, the anti-neurotonin antiserum was diluted in 1/1,000 with TBS (Tris Buffered Saline) which was added by 2.0mg/mL GST-short neurotonin fused protein and 5% skimmed milk and allowed to give rise to an immunoreaction at a room temperature for 60 minutes. Moreover, an experiment for allowing the same antibody solution to react with the NC membrane only as a negative control or an experiment for exchanging the GST-short neurotonin fused protein of the antibody solution for only GST was carried out at the same time. The NC membrane obtained after the reaction was washed with 0.1% Tween 20/TBS and then was subjected to an immunoreaction at a room temperature for 60 minutes with an HRP labeling anti-rabbit IgG antibody as a secondary antibody, and was washed with 0.1% Tween 20/TBS to detect an HRP activity so that a target antigen was detected. For the detection of the HRP activity, ECL (Amersham Co., Ltd.) was used (Clin. Chem., 25:1531, 1979). As a result, it was observed that the neurotonin is produced in an NB-1 cell.

### Example 4

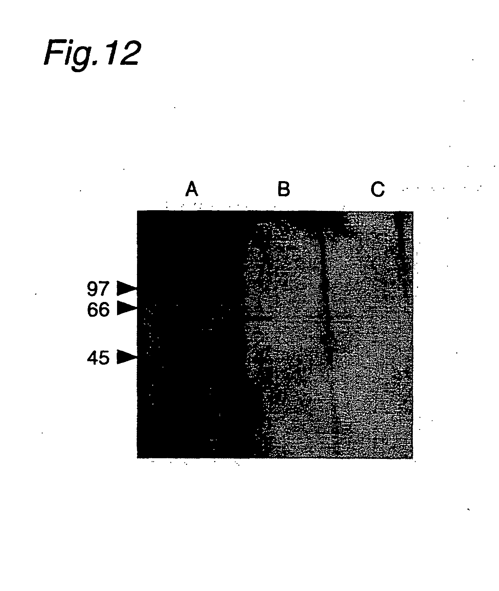

Detection of an anti-human neurotonin antibody in a serum of a patient (Fig. 12)

By using the GST-short neurotonin-fused protein prepared as described above as an antigen, an anti-neurotonin antibody presenting in a human serum was detected by a Western blotting method.

First of all, a GST-short neurotonin-fused protein (100ng/lane) was transferred to an NC membrane by SDS-PAGE electrophoresis. For a primary antibody were used sera obtained from (five) Isaacs syndrome patients, (thirty) patients having other peripheral nerve lesions other than the Isaacs syndrome who complained of the painful numbness of hands and legs as a complication of diabetes or the like, and (eight) people in normal health. In order to examine the presence of an anti-neurotonin antibody in the serum, a serum sample diluted in 1/1,000 with TBS was applied to the NC membrane to give rise to an immunoreaction at a room temperature for 60 minutes. The NC membrane was washed with 0.1% Tween 20/TBS and was then subjected to an immunoreaction at a room temperature for 60 minutes by using an HRP labeled anti-human IgG antibody as a secondary antibody. The NC membrane was washed with 0.1% Tween 20/TBS to detect an HRP activity. Thus, a human IgG reacting to a target antigen was detected. The HRP activity was measured by the above-mentioned method.

As a result, the anti-neurotonin antibody was detected from four of the five Isaacs syndrome patients and seven of the thirty patients having the peripheral nerve lesions other than the Isaacs syndrome, and was not detected from the (eight) people in normal health. Fig. 11 shows a part of the results.

Referring to the sera of the patients from which the antibody was detected, an antibody directed to the C terminal region of the neurotonin was detected from the four positive Isaacs syndrome patients and was similarly detected from the seven positive patients having the peripheral nerve lesions other than the Isaacs syndrome, and furthermore, an antibody directed to the N terminal portion of the neurotonin was detected from two other people.

### Example 5

### Detection of a neurotonin encoding cDNA by hybridization cloning of a mouse genome

As a library, Mouse Genomic Library (Clontech Co., Ltd.) derived from a mouse liver was used and the full length sequence of the human neurotonin cDNA was employed for a probe. The probe was labeled with a radioisotope (³²P) by using Bca BEST Labeling Kit (Takara Co., Ltd.). Phage DNAs per plate were transferred to two sheets of GeneScreen Plus (Du Pont Co., Ltd.), and a hybridization was carried out overnight with the labeled probe in a reaction solution (1M NaCl 1% SDS (Sodium Dodecyl Sulfate), 10% Dextran, 100g/ml salmon sperm DNA). After the hybridization, the filter was washed twice at a room temperature with 2 X SSC (an abbreviation of Standard Saline Citrate which implies 0.15M NaCl and 0.015M sodium citrate pH 7.2), twice at 65°C with 2 X SSC / 1% SDS, and twice at the room temperature with 0.1 X SSC, and a positive plague was then detected by autoradiography. Under the same conditions, second and third screening operations were carried out to detect and isolate the positive plague by using an imaging analyzer (Fujix Co., Ltd.).

The phage DNA used in the analysis was purified by using Wizard ™ Lambda Preps DNA Purification System (Promega Co., Ltd.). As a result, nine positive clones were obtained.

### Southern Blot Analysis

In order to examine that nine positive clones obtained by hybridization cloning contain a region including an ATG . equivalent to the methionine initiation codon, the following analysis was conducted.

1ng of a genome phage DNA thus purified was digested with a restriction enzyme SacI and was separated by 0.5% agarose electrophoresis. A gel is subjected to ethidium bromide staining and the genome DNA was confirmed to be digested, and a DNA in the gel was then transferred onto a filter overnight with an alkaline buffer (0.4M NaOH, 0.6M NaCl) by a blotting stone method. The filter neutralized with 50mM NaOH and 1M Tris-HCl (pH8.0) was air-dried and was then immersed in a prehybridization solution (1% SDS, 1M NaCl, 10% Dextran), and was thereafter subjected to the prehybridization at 65°C for 30 minutes.

Next, a probe to specifically recognize a region containing an ATG equivalent to the methionine initiation codon of the neurotonin cDNA and a flanking sequence thereof was labeled with a radioisotope (³²P) by using Bca BEST Labeling Kit (Takara Co., Ltd.). The probe and 100g/ml salmon sperm DNA were added to the aforesaid prehybridization solution to carry out a hybridization overnight at 65°C. After the hybridization was ended, the filter was washed twice at a room temperature with 2 X SSC, twice at 65°C with 2 X SSC / 1% SDS, and twice at the room temperature with 0.1 X SSC. Then, the filter was photosensitized into an imaging plate to detect and isolate positive plagues by using an imaging analyzer (Fujix Co., Ltd.).

As a result, it was found that a 5' region containing ATG corresponding to the methionine initiation codon is present in three of the nine clones. More specifically, approximately 2 kb of band was detected from one clone and approximately 7 kb of band was detected from two clones. These three clones are found to be genome DNAs containing a 5' upstream region.

### Example 6

### Isolation of a mouse cDNA

It is possible to obtain a cDNA encoding a mouse neurotonin by the hybridization cloning of a mouse genome in the following manner.

A mouse embryo derived Mouse Embryo Lambda cDNA Library (STRATAGENE Co., Ltd.) was used as a library and the full length sequence of the human neurotonin cDNA employed for a probe. The probe was labeled by using Bca BEST Labeling Kit (Takara Co., Ltd.) with a radioisotope (³²P).Phage DNAs per plate were transferred to two sheets of GeneScreen Plus (Du Pont Co., Ltd.), and was allowed to react with the labeled probe at 65°C for eight hours in a reaction solution (1M NaCl, 1% SDS, 10% Dextran, 100g/ml salmon sperm DNA). The filter was washed twice at a room temperature with 2 X SSC, twice at 65°C with 2 X SSC / 1% SDS, and twice at the room temperature with 0.1 X SSC, and a positive plague was then detected by autoradiography. Under the same conditions, second and third screening operations were carried out to detect and isolate positive plagues by using an imaging analyzer (Fujix Co., Ltd.).

The phage DNA used in the above analysis was purified by using Wizard ™ Lambda Preps DNA Purification System (Promega Co., Ltd.). As a result, one positive clone was obtained.

A genome phage DNA 1 thus purified was cleaved by restriction enzymes EcoRI and XhoI and an insert was then cut out. The insert was further subjected to subcloning, at EcoRI and XhoI sites of a cloning vector, pBluescriptII. Consequently, a cDNA encoding a mouse neurotonin could be obtained. In the analysis of the nucleotide sequence of the cDNA, the sequence was determined by an ABIPRISM377 DNA Sequencing System (Perkin Elmer Co., Ltd.).

The nucleotide sequence thus analyzed was converted into an amino acid sequence. Consequently, it was found that said sequence contains the methionine initiation codon and is homologous with the nucleotide sequence of a human neurotonin by 85% or more.

## Claims

1. A peripheral nerve cell protein having the following properties (which is hereinafter referred to as a "neurotonin"),
wherein the protein
a) is found in peripheral nerve cells of Isaacs syndrome patients or other patients having peripheral nerve lesions,
b) reacts with an antibody found in a blood of the patients,
c) causes a similar symptom to an Isaacs syndrome group with an immunization to a rabbit, and
d) has a molecular weight of approximately 66 kDa according to SDS-PAGE.

2. A cDNA encoding a neurotonin.

3. A DNA encoding a neurotonin or including all nucleotide sequences shown in Fig. 1 or 2.

4. A nucleic acid molecule encoding a neurotonin, including at least one nucleotide sequence substantially corresponding to a whole or part of the nucleotide sequences shown in Fig. 1 or 2, or including a sequence which is substantially homologous with any of the sequences or is hybridized with any of the sequences.

5. A mouse cDNA encoding a mouse neurotonin.

6. A DNA encoding a mouse neurotonin or including all nucleotide sequences shown in Fig. 3 or 4.

7. A nucleic acid molecule encoding a mouse neurotonin, including at least one nucleotide sequence substantially corresponding to a whole or part of the nucleotide sequences shown in Fig. 3 or 4, or including a sequence encoding the mouse neurotonin which is substantially homologous with any of the sequences or is hybridized with any of the sequences.

8. An antisense RNA or an antisense DNA to the cDNA according to claim 2 or 5 or the nucleic acid molecule according to claim 3, 4, 6 or 7.

9. A ribozyme recognizing and cleaving the cDNA according to claim 2 or 5, the nucleic acid molecule according to claim 3, 4, 6 or 7, or an RNA transcribed from a part thereof.

10. An expression vector, a cloning vector or a cosmid containing the nucleic acid molecule according to any of claims 2 to 8.

11. A transformant retaining the vector or the cosmid according to claim 10.

12. A prokaryotic cell, a eukaryotic cell or a variant cell which contains the nucleic acid molecule according to any of claims 2 to 8.

13. A neurotonin having an amino acid sequence shown in Fig. 5 or 6.

14. A protein defined in the following a) or b) which has an activity to bind to an antibody found in a blood of the patient:
a) a polypeptide such as a polypeptide having an amino acid sequence shown in Fig. 5 or 6, a polypeptide having an amino acid sequence in which at least one amino acid is deleted, substituted or added and/or inserted in the amino acid sequence shown in Fig. 5 or 6, a synthetic polypeptide including an amino acid sequence constituting an antigenic portion of a neurotonin, functionally equivalent variants thereof, or a fused polypeptide further including the aforesaid amino acid sequences; and
b) a protein encoded by a DNA to be hybridizable with the DNA having the nucleotide sequence according to any of claims 2 to 4.

15. A protein defined in the following a) or b) which can bind to a "14-3-3" protein existing in a peripheral nerve cell:
a) a polypeptide such as a polypeptide having an amino acid sequence shown in Fig. 5 or 6, a polypeptide having an amino acid sequence in which at least one amino acid is deleted, substituted or added and/or inserted in the amino acid sequence shown in Fig. 5 or 6, a synthetic polypeptide including an amino acid sequence constituting an antigenic portion of a neurotonin, functionally equivalent variants thereof, or a fused polypeptide further including the aforesaid amino acid sequences; and
b) a protein encoded by a DNA to be hybridizable with the DNA having the nucleotide sequence according to any of claims 2 to 4.

16. An immunologically active domain of the protein according to any of claims 13 to 15 or a fragment having the domain.

17. A polypeptide such as a polypeptide having an amino acid sequence shown in Fig. 7 or 8, a polypeptide having an amino acid sequence in which at least one amino acid is deleted, substituted or added and/or inserted in the amino acid sequence shown in Fig. 7 or 8, a synthetic polypeptide including an amino acid sequence constituting an antigenic portion of a neurotonin, functionally equivalent variants thereof, or a fused polypeptide further including the aforesaid amino acid sequences.

18. A reagent comprising the protein according to any of claims 13 to 17 for an immunological analysis to assay an antibody recognizing the protein.

19. The reagent for an immunological analysis according to claim 18 for use to assess a diagnosis or treatment effect of an Isaacs syndrome or other peripheral nerve lesions.

20. A reagent comprising an antibody to react with the protein according to any of claims 13 to 17 for an immunological analysis to assay the protein.

21. The reagent for an immunological analysis according to claim 20 for use to assess a diagnosis or treatment effect of an Isaacs syndrome or other peripheral nerve lesions.

22. The reagent for an immunological analysis according to claim 21, wherein the protein according to any of claims 13 to 17 to be analyzed is present in peripheral nerve cells.

23. A neurotonin detecting method of analyzing an antibody which is present in a body fluid of a subject and reacts with the protein according to any of claims 13 to 17.

24. A method of screening a ligand to bind to the protein according to any of claims 13 to 17, comprising the steps of:
a) Causing a candidate compound of the ligand to come in contact with the protein according to any of claims 13 to 17; and
b) selecting a candidate compound having a binding activity to the protein.

25. An assay method of the protein according to any of claims 13 to 17 on the basis of an affinity of an antiligand and the ligand obtainable by the method according to claim 24 by using, as the antiligand, the protein.

26. A screening method of a compound inhibiting a binding of the protein according to any of claims 13 to 17 to the ligand, comprising the steps of:
a) causing the protein according to any of claims 13 to 17 to come in contact with the ligand under the presence of a candidate compound; and
b) selecting a candidate compound having an activity to inhibit the binding of the protein to the ligand.

27. A compound which can be obtained by the screening method according to claim 26.

28. A neurotonin inhibitor comprising a compound which can be obtained by the screening method according to claim 26.

29. A medicine for treating peripheral nerve lesions, comprising a compound which can be obtained by the screening method according to claim 26.

30. An antibody binding to the protein according to any of claims 13 to 17.

31. The antibody according to claim 30, wherein the antibody is a monoclonal antibody.

32. A monoclonal antibody against an antibody causing peripheral nerve lesions.

33. A method of detecting or separating a cell expressing the protein according to any of claims 13 to 17 by setting, as a marker, the protein or an expression of a gene encoding the protein.

34. The method according to claim 33, wherein the cell is a peripheral nerve cell.

35. A reagent for detecting or separating a cell expressing the protein according to any of claims 13 to 17,comprising the antibody according to any of claims 30 to 32.

36. A reagent for detecting the DNA according to claims 2 to 7 or an RNA transcribed therefrom, comprising a nucleic acid molecule having a nucleotide sequence which can hybridize with the DNA or a part thereof, or the RNA.

37. A transgenic non-human vertebrate in which an expression of the DNA according to claim 3 or 6 is modified or inducible the modification.

38. The animal according to claim 37 which is a peripheral nerve lesion model animal.

39. The knockout non-human vertebrate according to claim 37,
wherein an expression of the intrinsic DNA according to claim 3 or 6 is suppressed.

40. The non-human vertebrate according to claim 37, wherein another gene is knocked out.

41. A cell derived from the non-human vertebrate according to any of claims 37 to 40.

42. A method of screening a compound to increase or reduce an activity of an intrinsic promoter of the DNA according to claim 3 or 6, comprising the steps of:
a) detecting an expression of a gene bound to a downstream region of the intrinsic promoter of the DNA according to claim 3 or 6 under the presence of a compound to be tested; and
b) selecting the compound to increase or reduce the expression.

43. A method of screening a compound to increase or reduce an activity of an intrinsic promoter of the DNA according to claim 3 or 6, comprising the steps of:
a) applying a compound to be tested to the non-human vertebrate according to claims 37 to 40 or a cell derived from the vertebrate; and
b) selecting the compound to increase or reduce an expression of a gene which is knocked in.

44. An animal immunized with a neurotonin which is to be used for studies of an Isaacs syndrome or other peripheral nerve lesions and for screening of a substance having an improvement action of the Isaacs syndrome or other peripheral nerve lesions.

45. A rabbit immunized with a neurotonin for screening a protein or a non-protein substance which improves a suppressed VGKC function, a signal transduction disorder in a nerve terminal or a painful muscular convulsion.

46. A diagnostic marker for detecting a specific binding to the monoclonal antibody by using, as a marker, a neurotonin, a related protein thereto or other antibodies causing peripheral nerve lesions, thereby measuring a level of a neurotonin, a related protein thereto or an antibody causing peripheral nerve lesions in a sample obtained from a subject in order to diagnose an Isaacs syndrome or other peripheral nerve lesions.

47. An examination method of offering data for diagnosing an Isaacs syndrome or other peripheral nerve lesions, wherein a level of a neurotonin or an antibody causing peripheral nerve lesions in a sample obtained from a subject is measured.

48. A blood filtering material for use in eliminating a numbness caused by the presence of a neurotonin or an antibody causative peripheral nerve lesions to carry out a treatment which allows a neurotonin antibody or an antibody causing peripheral nerve lesions in a blood to bind to the monoclonal antibody fixed onto the filtering material through a dialytic filtration of the blood, thereby reducing the neurotonin antibody or the antibody causative the peripheral nerve lesions in a body.

49. A vaccine composition for stimulating an immune response to an Isaacs syndrome or other peripheral nerve lesions in a human being or other animals which comprises at least one polypeptide according to any of claims 13 to 17 together with a pharmaceutically acceptable carrier and stimulates an immune response against the polypeptide, or which comprises a virus cell or a host cell having the nucleic acid molecule according to any of claims 2 to 8 which is inserted in the cell and stimulates an immune response against a polypeptide encoded by the inserted nucleic acid molecule.

50. A cell culture medium comprising the monoclonal antibody according to claim 31 or 32 for in vitro reproducing a cell to be a peripheral nerve by removing an antibody causative peripheral nerve lesions through an incubation with peripheral nerve and muscle system cells separated from a patient having peripheral nerve lesions, to thereby cause a specific binding of the causative antibody to the monoclonal antibody.


SEQUENCE LISTINGS

<110> LOCOMOGENE, INC.

<120> NEUROTONIN AND ITS USE

<130>

<140> JAPAN, 2001-34217
<141> 2001-02-09

<160> 4

<170>

<210> 1
<211> 1347
<212> cDNA
<213> human

<220>
<223> Short cDNA for neurotonin

<400> 1
tgacagaagg gagaagaaag aaatgaagcg agttttcagt attccaagca taagagccag 60
caagatacat tccctcaagt gtccagaatt tccaattaca gacgacaaag tagcactgat 120
tcgaattcag aattgtcaaa tgaagaatta aggcaatgtc ttaatgaaac tttagaggag 180
gtagaa atg tta aaa act gaa ctt gag gca tct caa aga caa ctc aga ggt aaa 234
Met Leu Lys Thr Glu Leu Glu Ala Ser Gln Arg Gln Leu Arg Gly Lys
1               5                   10                  15
gag gaa gca ttg aaa att ctt caa agc atg gca ata ctg ggc aaa gcc 282
Glu Glu Ala Leu Lys Ile Leu Gln Ser Met Ala Ile Leu Gly Lys Ala
            20                  25                  30
aca agt cat acg cag gca gtg ctt caa aaa act atg gaa caa aac aga 330
Thr Ser His Thr Gln Ala Val Leu Gln Lys Thr Met Glu Gln Asn Arg
        35                  40                  45
tcc ttg gag aag gaa ata aat gcc ttg cag tgg gaa ata gaa ttt gat 378
Ser Leu Glu Lys Glu Ile Asn Ala Leu Gln Trp Glu Ile Glu Phe Asp
    50                  55                  60
cat aat aga ttt aaa aat ata gag gaa tct tgg atc caa aaa tat gac 426
His Asn Arg Phe Lys Asn Ile Glu Glu Ser Trp Ile Gln Lys Tyr Asp
65                  70                  75                  80
agg cta aac tgt gaa aat gca gtc ctc aaa gag aat ttg aaa gtg aaa 474
Arg Leu Asn Cys Glu Asn Ala Val Leu Lys Glu Asn Leu Lys Val Lys
                85                  90                  95
aca gaa gaa att aaa atg ctg aag tct gac aat gca gtt ttg aat caa 522
Thr Glu Glu Ile Lys Met Leu Lys Ser Asp Asn Ala Val Leu Asn GLn
            100                 105                 110
cgg tat ttg gag gcc ctc gcc atg ctt gat atc aaa cag cag aag atg 570
Arg Tyr Leu Glu Ala Leu Ala Met Leu Asp Ile Lys Gln Gln Lys Met
        115                 120                 125
gct cag gaa aac atg tgc tgt gat aaa agt ggc ttt gca gag gct tca 618
Ala Gln Glu Asn Met Cys Cys Asp Lys Ser Gly Phe Ala Glu Ala Ser
    130                 135                 140
ggt ctt gag ctt gcg gtc ctc gga gcc tgc ctt tgt cat ggg ccc gga 666
Gly Leu Glu Leu Ala Val Leu Gly Ala Cys Leu Cys His Gly Pro Gly
145                 150                 155                 160
ggg aac ccc tgt tct tgt gcc aga atg gca gca tcc act cgg aaa ctg 714
Gly Asn Pro Cys Ser Cys Ala Arg Met Ala Ala Ser Thr Arg Lys Leu
                165                 170                 175

ctt ctt cag ctc aaa caa gag ttg gaa att ttg cag aag agt aaa gaa 762
Leu Leu Gln Leu Lys Gln Glu Leu Glu Ile Leu Gln Lys Ser Lys Glu
            180                 185                 190
gaa gct tac gtg atg gca gat gct ttc aga att gca ttt gag caa caa 810
Glu Ala Tyr Val Met Ala Asp Ala Phe Arg Ile Ala Phe Glu Gln Gln
        195                 200                 205
tta atg aga aaa aat gac cag gca cta caa ttg aca caa atg gat aaa 858
Leu Met Arg Lys Asn Asp Gln Ala Leu Gln Leu Thr Gln Met Asp Lys
    210                 215                 220
atg cat aaa aaa gca aca aaa tgg atg aat tgg aag cac ctt aaa gag 906
Met His Lys Lys Ala Thr Lys Trp Met Asn Trp Lys His Leu Lys Glu
225                 230                 235                 240
gat gga ttt cca tca cca agg agt aag aag acc ttc ggg cag aga ctg 954
Asp Gly Phe Pro Ser Pro Arg Ser Lys Lys Thr Phe Gly Gln Arg Leu
                245                 250                 255
ttg ggt atg ctc cct tca gaa aac agt tct aag agg atg gaa gac cag 1002
Leu Gly Met Leu Pro Ser Glu Asn Ser Ser Lys Arg Met Glu Asp Gln
            260                 265                 270
gac agt cct caa gag gtc ctt aag atg ctc ata gat ttg ctt aat gat 1050
Asp Ser Pro Gln Glu Val Leu Lys Met Leu Ile Asp Leu Leu Asn Asp
        275                 280                 285
aaa gaa gaa gct ttg gct cat caa aga aaa gtt agc tac atg ctt gct 1098
Lys Glu Glu Ala Leu Ala His Gln Arg Lys Val Ser Tyr Met Leu Ala
    290                 295                 300
cgg gca ttg gaa gac aaa gac act gct tca aac gag aat aaa gaa aaa 1146
Arg Ala Leu Glu Asp Lys Asp Thr Ala Ser Asn Glu Asn Lys Glu Lys
305                 310                 315                 320
aat cct ata aaa gag aat ttc cct ttc aac aac ccc tgg cgt aag act 1194
Asn Pro Ile Lys Glu Asn Phe Pro Phe Asn Asn Pro Trp Arg Lys Thr
                325                 330                 335
tca gaa ttc tct gtt ttg ggt gat cct ata cat tca agt gtc tgc att 1242
Ser Glu Phe Ser Val Leu Gly Asp Pro Ile His Ser Ser Val Cys Ile
            340                 345                 350
tta aat tct gtg ggc tgc att tgt tca atc cag cac tct caa ata gat 1290
Leu Asn Ser Val Gly Cys Ile Cys Ser Ile Gln His Ser Gln Ile Asp
        355                 360                 365
cca aac tat aga act ctt aaa aga tcc cat tct ttg cca tca agt atc 1338
Pro Asn Tyr Arg Thr Leu Lys Arg Ser His Ser Leu Pro Ser Ser Ile
    370                 375                 380
ata ttt taa 1347
Ile Phe
385

<210> 2
<211> 2738
<212> cDNA
<213> human

<220>
<223> Long cDNA for neurotonin

<400> 2
gagctggagt ccgggctccg gtcccctcca cggaccttga gagggtaccc ggggtcagac 60
ctggcagaca gcccattttt tcttatgata aagacggcat ttggctc 107

atg agc aag gtg gca aga tca tca agt gag tca gac atg cag ctc tgg 155
Met Ser Lys Val Ala Arg Ser Ser Ser Glu Ser Asp Met Asn Leu Trp
1               5                   10                  15
gaa aca gaa gag gat gac atg aca gaa ggt gat tta ggg tat ggc ctc 203
Glu Thr Glu Glu Asp Asp Met Thr Glu Gly Asp Leu Gly Tyr Gly Leu
            20                  25                  30

gga agg aaa cct ggt ggg att tat gaa ata gaa ttt tca cat agg tct 251
Gly Arg Lys Pro Gly Gly Ile Tyr Glu Ile Glu Phe Ser His Arg Ser
        35                  40                  45
aga aaa aga tca gat gga aag aac ttt agc cct cct cca ttt ccg aga 299
Arg Lys Arg Ser Asp Gly Lys Asn Phe Ser Pro Pro Pro Phe Pro Arg
    50                  55                  60
aag gga gaa gaa aga aat gaa gcg agt ttt cag tat tcc aag cat aag 347
Lys Gly Glu Glu Arg Asn Glu Ala Ser Phe Asn Tyr Ser Lys His Lys
65                  70                  75                  80
agc cag caa gat aca ttc cct caa gtg tcc aga att tcc aat tac aga 395
Ser Gln Gln Asp Thr Phe Pro Gln Val Ser Arg Ile Ser Asn Tyr Arg
                85                  90                  95
cga caa agt agc act gta gat tcg aat tca gaa ttg tca aat gaa gaa 443
Arg Gln Ser Ser Thr Val Asp Ser Asn Ser Glu Leu Ser Asn Glu Glu
            100                 105                 110
tta agg caa tgt ctt aat gaa act tta gag gag gta gaa atg tta aaa 491
Leu Arg Gln Cys Leu Asn Glu Thr Leu Glu Glu Val Glu Met Leu Lys
        115                 120                 125
act gaa ctt gag gca tct caa aga caa ctc aga ggt aaa gag gaa gca 539
Thr Glu Leu Glu Ala Ser Gln Arg Gln Leu Arg Gly Lys Glu Glu Ala
    130                 135                 140
ttg aaa att ctt caa agc atg gca ata ctg ggc aaa gcc aca agt cat 587
Leu Lys Ile Leu Gln Ser Met Ala Ile Leu Gly Lys Ala Thr Ser His
145                 150                 155                 160
acg cag gca gtg ctt caa aaa act atg gaa caa aac aga tcc ttg gag 635
Thr Gln Ala Val Leu Gln Lys Thr Met Glu Gln Asn Arg Ser Leu Glu
                165                 170                 175
aag gaa ata aat gcc ttg cag tgg gaa ata gaa ttt gat cat aat aga 683
Lys Glu Ile Asn Ala Leu Gln Trp Glu Ile Glu Phe Asp His Asn Arg
            180                 185                 190
ttt aaa aat ata gag gaa tct tgg atc caa aaa tat gac agg cta aac 731
Phe Lys Asn Ile Glu Glu Ser Trp Ile Gln Lys Tyr Asp Arg Leu Asn
        195                 200                 205
tgt gaa aat gca gtc ctc aaa gag aat ttg aaa gtg aaa aca gaa gaa 779
Cys Glu Asn Ala Val Leu Lys Glu Asn Leu Lys Val Lys Thr Glu Glu
    210                 215                 220
att aaa atg ctg aag tct gac aat gca gtt ttg aat caa cgg tat ttg 827
Ile Lys Met Leu Lys Ser Asp Asn Ala Val Leu Asn Gln Arg Tyr Leu
225                 230                 235                 240
gag gcc ctc gcc atg ctt gat atc aaa cag cag aag atg gct cag gaa 875
Glu Ala Leu Ala Met Leu Asp Ile Lys Gln Gln Lys Met Ala Gln Glu
                245                 250                 255
aac atg tgc tgt gat aaa agt ggc ttt gca gag gct tca ggt ctt gag 923
Asn Met Cys Cys Asp Lys Ser Gly Phe Ala Glu Ala Ser Gly Leu Glu
            260                 265                 270
ctt gcg gtc ctc gga gcc tgc ctt tgt cat ggg ccc gga ggg aac ccc 971
Leu Ala Val Leu Gly Ala Cys Leu Cys His Gly Pro Gly Gly Asn Pro
        275                 280                 285
tgt tct tgt gcc aga atg gca gca tcc act cgg aaa ctg ctt ctt cag 1019
Cys Ser Cys Ala Arg Met Ala Ala Ser Thr Arg Lys Leu Leu Leu Gln
    290                 295                 300
ctc aaa caa gag ttg gaa att ttg cag aag agt aaa gaa gaa gct tac 1067
Leu Lys Gln Glu Leu Glu Ile Leu Gln Lys Ser Lys Glu Glu Ala Tyr
305                 310                 315                 320
gtg atg gca gat gct ttc aga att gca ttt gag caa caa tta atg aga 1115
Val Met Ala Asp Ala Phe Arg Ile Ala Phe Glu Gln Gln Leu Met Arg
                325                 330                 335
aaa aat gac cag gca cta caa ttg aca caa atg gat aaa atg cat aaa 1163
Lys Asn Asp Gln Ala Leu Gln Leu Thr Gln Met Asp Lys Met His Lys
            340                 345                 350
aaa gca aca aaa tgg atg aat tgg aag cac ctt aaa gag gat gga ttt 1211

Lys Ala Thr Lys Trp Met Asn Trp Lys His Leu Lys Glu Asp Gly Phe
        355                 360                 365
cca tca cca agg agt aag aag acc ttc ggg cag aga ctg ttg ggt atg 1259
Pro Ser Pro Arg Ser Lys Lys Thr Phe Gly Gln Arg Leu Leu Gly Met
    370                 375                 380
ctc cct tca gaa aac agt tct aag agg atg gaa gac cag gac agt cct 1307
Leu Pro Ser Glu Asn Ser Ser Lys Arg Met Glu Asp Gln Asp Ser Pro
385                 390                 395                 400
caa gag gtc ctt aag atg ctc ata gat ttg ctt aat gat aaa gaa gaa 1355
Gln Glu Val Leu Lys Met Leu Ile Asp Leu Leu Asn Asp Lys Glu Glu
                405                 410                 415
gct ttg gct cat caa aga aaa gtt agc tac atg ctt gct cgg gca ttg 1403
Ala Leu Ala His Gln Arg Lys Val Ser Tyr Met Leu Ala Arg Ala Leu
            420                 425                 430
gaa gac aaa gac act gct tca aac gag aat aaa gaa aaa aat cct ata 1451
Glu Asp Lys Asp Thr Ala Ser Asn Glu Asn Lys Glu Lys Asn Pro Ile
        435                 440                 445
aaa gag aat ttc cct ttc aac aac ccc tgg cgt aag act tca gaa ttc 1499
Lys Glu Asn Phe Pro Phe Asn Asn Pro Trp Arg Lys Thr Ser Glu Phe
    450                 455                 460
tct gtt ttg ggt gat cct ata cat tca agt gtc tgc att tta aat tct 1547
Ser Val Leu Gly Asp Pro Ile His Ser Ser Val Cys Ile Leu Asn Ser
465                 470                 475                 480
gtg ggc tgc att tgt tca atc cag cac tct caa ata gat cca aac tat 1595
Val Gly Cys Ile Cys Ser Ile Gln His Ser Gln Ile Asp Pro Asn Tyr
                485                 490                 495
aga act ctt aaa aga tcc cat tct ttg cca tca agt atc ata ttt 1640
Arg Thr Leu Lys Arg Ser His Ser Leu Pro Ser Ser Ile Ile Phe
            500                 505                 510

taaagacaag ccagttgaaa ttggaactga gagttgttta 1680
tatcgagata ctttgaaaaa tgattttgta aattttgctg catcttgaga agttgtatgt 1740
ttcctaggta tttctaaatt ttaggaggtc acttaaatca aatattttct acattttctt 1800
tttcttcttt tgagatggag tcttgctctg tcacccaggt tggagtgcag tggtgtgatc 1860
tcggttcact gcaacctccg cctcccggtt caggcaattc tcctgcctca gcctcccgag 1920
tgactcggat tacaggcatg tgccatctct actaaaaata caaaaaattg gctgggccta 1980
gtggcgcatg cctgtaatcc cagctgcttg ggaggctgag gtaggagagt cgcttgagcc 2040
tgggaggcgg gggttgtggt gagccgagac tgtgtcattg cactccagcc tgggcgacaa 2100
gagcgaaact ctgtctcaaa aaaaaaaaaa aaacacatta cagtgtttgc caacatccta 2160
aaactaaaaa ataatgattt ttgggaaaaa caaaaaatta tttaactatt ttgattgagt 2220
atagggatct tagcttggct aaatcaccaa tatagcaatg tatcttaatg ctctaaaaag 2280
taacaatacc taggactgac tgaatgttaa tttctggaat tcatttatct tgttttattt 2340
gccttgttca gtatttttca tttaccatat gaagtttaga aataacaatt tccttaagtt 2400
aacttcaata agacagtttt gatggtggga ccttggcaga agtatattga gtcagtgccg 2460
gctgcgttca tggcttcttg tcagggtagt aacattttgc aaccaaatct gcacagattt 2520
gtagccaagt ctacaaagtg cttaacagct cttttcattg catcctgtga gatggttgtg 2580
atgatccagg aaatggggtc tcaggaatct gaagaagctg cctgagccaa ggtgatttct 2640
aggagttttt ttgtttgtgg tatgtgaatt ggggtgtgtg ggggtggggt gtgtgtgtag 2700
acagggtccc actgtgttgc tcaggctggt gccaaact 2738

<210> 3
<211> 1326
<212> cDNA
<213> mouse

<220>
<221> unsure
<222> 917,919,922,924
<223> Short cDNA for neurotonin

<400> 3

tgaccgaagg gagaagaaag aagcgaaacc agtttccagt attccaggag gaagggcttt 60
caagatacaa gcgctgaggg ataccgagca tccagactga gcagcacaga ttctaactca 120
gaattgtcgg atgagcagtt aaggcgacgt cttcatgaag ccttagagga cgtagagatt 180
ttaaaaacgg aacttgaagc gtctcaaaga caacttgaag gtaaagagga agcattgaaa 240
atcctccaaa gc 252
atg gca atg ctt ggc aaa gcc aca agc cac aca cag aca atg ctt caa 300
Met Ala Met Leu Gly Lys Ala Thr Ser His Thr Gln Thr Met Leu Gln
1               5                   10                  15
aaa act ata gaa caa aag aga tct ctg gag aag gaa ata aat gcc ttg 348
Lys Thr Ile Glu Gln Lys Arg Ser Leu Glu Lys Glu Ile Asn Ala Leu
            20                  25                  30
cag tgg gaa atg gaa ttt gat cag gat aga ttt aaa aat ata gaa gaa 396
Gln Trp Glu Met Glu Phe Asp Gln Asp Arg Phe Lys Asn Ile Glu Glu
        35                  40                  45
tct tgg atc cag aaa tgt gac agg cta aac tgt gac aat gca gtc ctc 444
Ser Trp Ile Gln Lys Cys Asp Arg Leu Asn Cys Asp Asn Ala Val Leu
    50                  55                  60
aga gag aat ctg aag ttg aga aca gag gaa ata aag atg cta aag tct 492
Arg Glu Asn Leu Lys Leu Arg Thr Glu Glu Ile Lys Met Leu Lys Ser
65                  70                  75                  80
aag aat gct gtt ttg aat cag cgg tac ttg gag gcc ctc gcc atg ctt 540
Lys Asn Ala Val Leu Asn Gln Arg Tyr Leu Glu Ala Leu Ala Met Leu
                85                  90                  95
gat atc aag gag cag aag atg ggt cag gag gag agt ggc ttt aca gat 588
Asp Ile Lys Glu Gln Lys Met Gly Gln Glu Glu Ser Gly Phe Thr Asp
            100                 105                 110
gta tca ggt ctc gag ctt gca gtc ctt gga gcc tgc ctg tgt cat ggt 636
Val Ser Gly Leu Glu Leu Ala Val Leu Gly Ala Cys Leu Cys His Gly
        115                 120                 125
cct gga ggg agc ccc tgt tct tgt gcc aaa atg gca gca tcc act cgg 684
Pro Gly Gly Ser Pro Cys Ser Cys Ala Lys Met Ala Ala Ser Thr Arg
    130                 135                 140
aaa ctg gtt ctt cag ctc aga cat gag ttg gaa act ctg cag aag agt 732
Lys Leu Val Leu Gln Leu Arg His Glu Leu Glu Thr Leu Gln Lys Ser
145                 150                 155                 160
aag gaa gag gcg cac ata acg gca gat gca ttc agg att gct ttt gag 780
Lys Glu Glu Ala His Ile Thr Ala Asp Ala Phe Arg Ile Ala Phe Glu
                165                 170                 175
caa cag tta atg agg aaa aat gag cag gca ctg aga ctg gct gga ggg 828
Gln Gln Leu Met Arg Lys Asn Glu Gln Ala Leu Arg Leu Ala Gly Gly
            180                 185                 190
gac ctg tgt aaa aaa gcg gca acc gtg gat caa cag aca aca ccc agg 876
Asp Leu Cys Lys Lys Ala Ala Thr Val Asp Gln Gln Thr Thr Pro Arg
        195                 200                 205
cag acg atg gat atc cgg cac aaa gga gaa aga aga cct tnt ngg ntn 924
Gln Thr Met Asp Ile Arg His Lys Gly Glu Arg Arg Pro Xaa Xaa Xaa
    210                 215                 220
aga tta ctg ggg ata ctc cct tcg gaa aac agc tcc aag ggc gct gaa 972
Arg Leu Leu Gly Ile Leu Pro Ser Glu Asn Ser Ser Lys Gly Ala Glu
225                 230                 235                 240
gac caa gac aat atg caa gag gtc ttt aag atg ctg gta gat ttg ttg 1020
Asp Gln Asp Asn Met Gln Glu Val Phe Lys Met Leu Val Asp Leu Leu
                245                 250                 255
aat gac aaa gaa gaa gcc ctt gca cat cag aga aag gtt agt tac atg 1068
Asn Asp Lys Glu Glu Ala Leu Ala His Gln Arg Lys Val Ser Tyr Met
            260                 265                 270
ctc gct cgg gcg ctg gaa gac aaa gac acg gcc tca gaa agg aat aaa 1116
Leu Ala Arg Ala Leu Glu Asp Lys Asp Thr Ala Ser Glu Arg Asn Lys
        275                 280                 285
gaa aaa atc ccc atg agc cag acc ttc cca ttc aaa acg gcc tgg cac 1164
Glu Lys Ile Pro Met Ser Gln Thr Phe Pro Phe Lys Thr Ala Trp His

290                 295                 300
gac gct tca gag ctc tgt ggt ctg cgt gat cca gta cag tca aac cat          1212
Asp Ala Ser Glu Leu Cys Gly Leu Arg Asp Pro Val Gln Ser Asn His
305                 310                 315                 320
gtt tct gaa ccc atg gct tgc atc tgt tca ata cag cat cct cca aaa          1260
Val Ser Glu Pro Met Ala Cys Ile Cys Ser Ile Gln His Pro Pro Lys
                325                 330                 335
gtt tca gac tgc cca aga act ctt aaa aga tcc tgt tct ttg cca tca          1308
Val Ser Asp Cys Pro Arg Thr Leu Lys Arg Ser Cys Ser Leu Pro Ser
            340                 345                 350
act tta ttt tac aag taa                                                  1326
Thr Leu Phe Tyr Lys
        355

<210> 4
<211> 1559
<212> cDNA
<213> mouse

<220>
<223> Long cDNA for neurotonin

<400> 4
tgagggagga ggctgagtgt ccagcagctc cctgggaccg acatttggct a                 51
atg agc aaa gtg ccg agg tca tcg agc gag gca gag gac atc tgg gaa          99
Met Ser Lys Val Pro Arg Ser Ser Ser Glu Ala Glu Asp Ile Trp Glu
  1               5                  10                  15
aca gag gat gac atg acc gaa ggt gac cta ggc tat ggc ctc gga aga          147
Thr Glu Asp Asp Met Thr Glu Gly Asp Leu Gly Tyr Gly Leu Gly Arg
             20                  25                  30
aaa ccc ggt ggt att tat gaa gtt ccg tgt tcg att aca tct aag aaa          195
Lys Pro Gly Gly Ile Tyr Glu Val Pro Cys Ser Ile Thr Ser Lys Lys
         35                  40                  45
agg tca gat gga aag aac tca agc cct cct ccg ttt cca aga aag gga          243
Arg Ser Asp Gly Lys Asn Ser Ser Pro Pro Pro Phe Pro Arg Lys Gly
     50                  55                  60
gaa gaa aga agc gaa acc agt ttc cag tat tcc agg agg aag ggc ttt          291
Glu Glu Arg Ser Glu Thr Ser Phe Gln Tyr Ser Arg Arg Lys Gly Phe
 65                  70                  75                  80
caa gat aca agc gct gag gga tac cga gca tcc aga ctg agc agc aca          339
Gln Asp Thr Ser Ala Glu Gly Tyr Arg Ala Ser Arg Leu Ser Ser Thr
                 85                  90                  95
gat tct aac tca gaa ttg tcg gat gag cag tta agg cga cgt ctt cat          387
Asp Ser Asn Ser Glu Leu Ser Asp Glu Gln Leu Arg Arg Arg Leu His
            100                 105                 110
gaa gcc tta gag gac gta gag att tta aaa acg gaa ctt gaa gcg tct          435
Glu Ala Leu Glu Asp Val Glu Ile Leu Lys Thr Glu Leu Glu Ala Ser
        115                 120                 125
caa aga caa ctt gaa ggt aaa gag gaa gca ttg aaa atc ctc caa agc          483
Gln Arg Gln Leu Glu Gly Lys Glu Glu Ala Leu Lys Ile Leu Gln Ser
    130                 135                 140
atg gca atg ctt ggc aaa gcc aca agc cac aca cag aca atg ctt caa          531
Met Ala Met Leu Gly Lys Ala Thr Ser His Thr Gln Thr Met Leu Gln
145                 150                 155                 160
aaa act ata gaa caa aag aga tct ctg gag aag gaa ata aat gcc ttg          579
Lys Thr Ile Glu Gln Lys Arg Ser Leu Glu Lys Glu Ile Asn Ala Leu
                165                 170                 175
cag tgg gaa atg gaa ttt gat cag gat aga ttt aaa aat ata gaa gaa          627
Gln Trp Glu Met Glu Phe Asp Gln Asp Arg Phe Lys Asn Ile Glu Glu
            180                 185                 190
tct tgg atc cag aaa tgt gac agg cta aac tgt gac aat gca gtc ctc          675

Ser Trp Ile Gln Lys Cys Asp Arg Leu Asn Cys Asp Asn Ala Val Leu
        195                 200                 205
aga gag aat ctg aag ttg aga aca gag gaa ata aag atg cta aag tct 723
Arg Glu Asn Leu Lys Leu Arg Thr Glu Glu Ile Lys Met Leu Lys Ser
    210                 215                 220
aag aat gct gtt ttg aat cag cgg tac ttg gag gcc ctc gcc atg ctt 771
Lys Asn Ala Val Leu Asn Gln Arg Tyr Leu Glu Ala Leu Ala Met Leu
225                 230                 235                 240
gat atc aag gag cag aag atg ggt cag gag gag agt ggc ttt aca gat 819
Asp Ile Lys Glu Gln Lys Met Gly Gln Glu Glu Ser Gly Phe Thr Asp
                245                 250                 255
gta tca ggt ctc gag ctt gca gtc ctt gga gcc tgc ctg tgt cat ggt 867
Val Ser Gly Leu Glu Leu Ala Val Leu Gly Ala Cys Leu Cys His Gly
            260                 265                 270
cct gga ggg agc ccc tgt tct tgt gcc aaa atg gca gca tcc act cgg 915
Pro Gly Gly Ser Pro Cys Ser Cys Ala Lys Met Ala Ala Ser Thr Arg
        275                 280                 285
aaa ctg gtt ctt cag ctc aga cat gag ttg gaa act ctg cag aag agt 963
Lys Leu Val Leu Gln Leu Arg His Glu Leu Glu Thr Leu Gln Lys Ser
    290                 295                 300
aag gaa gag gcg cac ata acg gca gat gca ttc agg att gct ttt gag 1011
Lys Glu Glu Ala His Ile Thr Ala Asp Ala Phe Arg Ile Ala Phe Glu
305                 310                 315                 320
caa cag tta atg agg aaa aat gag cag gca ctg aga ctg gct gga ggg 1059
Gln Gln Leu Met Arg Lys Asn Glu Gln Ala Leu Arg Leu Ala Gly Gly
3[?] 330 335
gac ctg tgt aaa [?] gcg gca acc gtg gat caa cag aca aca ccc agg 1107
Asp Leu Cys Lys Lys Ala Ala Thr Val Asp Gln Gln Thr Thr Pro Arg
340 345 350
cag acg atg gat atc cgg cta caa agg aga aag aag acc tta ggg caa 1155
Gln Thr Met Asp Ile Arg Leu Gln Arg Arg Lys Lys Thr Leu Gly Gln
355 360 365
aga tta ctg ggg ata ctc cct tcg gaa aac agc tcc aag ggc gct gaa 1203
Arg Leu Leu Gly Ile Leu Pro Ser Glu Asn Ser Ser Lys Gly Ala Glu
370 375 380
gac caa gac aat atg caa gag gtc ttt aag atg ctg gta gat ttg ttg 1251
Asp Gln Asp Asn Met Gln Glu Val Phe Lys Met Leu Val Asp Leu Leu
385 390 395 400
aat gac aaa gaa gaa gcc ctt gca cat cag aga aag gtt agt tac atg 1299
Asn Asp Lys Glu Glu Ala Leu Ala His Gln Arg Lys Val Ser Tyr Met
405 410 415
ctc gct cgg gcg ctg gaa gac aaa gac acg gcc tca gaa agg aat aaa 1347
Leu Ala Arg Ala Leu Glu Asp Lys Asp Thr Ala Ser Glu Arg Asn Lys
420 425 430
gaa aaa atc ccc atg agc cag acc ttc cca ttc aaa acg gcc tgg cac 1395
Glu Lys Ile Pro Met Ser Gln Thr Phe Pro Phe Lys Thr Ala Trp His
435 440 445
gac gct tca gag ctc tgt ggt ctg cgt gat cca gta cag tca aac cat 1443
Asp Ala Ser Glu Leu Cys Gly Leu Arg Asp Pro Val Gln Ser Asn His
450 455 460
gtt tct gaa ccc atg gct tgc atc tgt tca ata cag cat cct cca aaa 1491
Val Ser Glu Pro Met Ala Cys Ile Cys Ser Ile Gln His Pro Pro Lys
465 470 475 480
gtt tca gac tgc cca aga act ctt aaa aga tcc tgt tct ttg cca tca 1539
Val Ser Asp Cys Pro Arg Thr Leu Lys Arg Ser Cys Ser Leu Pro Ser
485 490 495
act tta ttt tac aag taa ac 1559
Thr Leu Phe Tyr Lys
500